# EUROPEAN PATENT APPLICATION

(11) **EP 4 834 878 A1**
(43) Date of publication of application: **04.11.2026**
(21) Application number: 24911152.7
(22) Date of filing: 25.12.2024
(51) Int. Cl.: C07C 231/12, C07C 237/06, C07C 309/04, A61K 31/165, A61P 25/16, A61P 25/28, A61P 25/08, A61P 25/24

(54) **AMIDE DERIVATIVE, PREPARATION METHOD THEREFOR AND USE THEREOF**

(30) Priority: 25.12.2023 CN 202311797362; 15.05.2024 CN 202410603964
(71) Applicant: CSPC Zhongqi Pharmaceutical Technology (Shijiazhuang) Co., Ltd., Shijiazhuang, Hebei 050035 (CN)
(72) Inventor: WANG, Zhenyu, Shijiazhuang, Hebei 050035 (CN); AN, Hui, Shijiazhuang, Hebei 050035 (CN); HUA, Xinxing, Shijiazhuang, Hebei 050035 (CN); ZHAO, Chuanwu, Shijiazhuang, Hebei 050035 (CN); SONG, Yunlong, Shijiazhuang, Hebei 050035 (CN); LIU, Xibao, Shijiazhuang, Hebei 050035 (CN); HAN, Yue, Shijiazhuang, Hebei 050035 (CN); TIAN, Jiahui, Shijiazhuang, Hebei 050035 (CN); WANG, Jia, Shijiazhuang, Hebei 050035 (CN); MA, Yuxiu, Shijiazhuang, Hebei 050035 (CN)
(74) Representative: Barrow, Nicholas Martin
(86) International application number: PCT/CN2024/142055
(87) International publication number: WO 2025/140228

(57) **Abstract**

The present invention provides an amide derivative shown as in formula (I) or a tautomer, stereoisomer or pharmaceutically acceptable salt thereof, and a use thereof. In-vitro experimental results show that the compound in the present invention has relatively high MAO-B selective inhibition activity. In-vivo experimental results show that the compound in the present invention can remarkably reduce the weight of a mouse under the condition that the feeding amount is not affected.

## Description

### TECHNICAL FIELD

The present invention relates to the field of medical technology, and in particular, to a novel class of amide derivatives, preparation methods therefor, and their pharmaceutical use as monoamine oxidase-B (MAO-B) inhibitors.

### BACKGROUND

Monoamine oxidase (MAO) is an enzyme that catalyzes the oxidative deamination of monoamines in the human body. The products of this reaction include corresponding aldehydes, amines and hydrogen peroxide. For example, hydrogen peroxide can trigger the production of reactive oxygen species (ROS) and induce mitochondrial damage and neuronal apoptosis. Depending on the substrate, monoamine oxidases are divided into two types: MAO-A and MAO-B. MAO-A exhibits high affinity for substrates such as tyramine, 5-hydroxytryptamine, adrenaline and noradrenaline. In contrast, MAO-B preferentially acts on dopamine, phenylethylamine and other trace arylalkylamines. MAO-B is expressed in a variety of organs, including the brain, heart, adipose tissue, pancreas, lungs, kidneys, muscles, and liver.

MAO-B is abundantly distributed in the basal ganglia of the brain, and its activity increases with age. Since MAO-B metabolizes dopamine (DA), it exacerbates DA deficiency in relevant brain regions of patients with Parkinson's disease (PD). Additionally, both aldehydes and hydrogen peroxide generated from MAO-B-catalyzed reactions exert neurotoxic effects. The deficiency of aldehyde dehydrogenase in the substantia nigra of PD patients further aggravates the neurotoxicity induced by MAO-B. Accordingly, MAO-B inhibition treats PD via two mechanisms: elevating the levels of monoamine neurotransmitters such as DA in the brain, and reducing neurotoxicity. Meanwhile, MAO-B-mediated redox damage is a prominent feature of Alzheimer's disease (AD), and evidence of ROS-mediated neuronal damage has been observed in the brains of AD patients. Therefore, in recent years, MAO-B has been considered a valuable potential target for the treatment of AD and has received widespread attention and research.

The complex balance between food intake and energy expenditure is controlled by the hypothalamus in the brain. Astrocytes in the lateral hypothalamus can regulate the activity of GABRA5 neurons. Studies have shown that as the number and volume of active astrocytes increase, they begin to overexpress MAO-B enzymes, which in turn produce large amounts of γ-aminobutyric acid (GABA), thereby inhibiting surrounding GABRA5 neurons, leading to reduced energy consumption and fat accumulation. Inhibiting the expression of the MAO-B gene in reactive astrocytes can reduce GABA secretion, thereby reversing the adverse inhibitory effect of GABRA5 neurons. Meanwhile, since neurons that suppress appetite do not express GABAa receptors outside the synapse, they are not affected by GABA. Therefore, MAO-B inhibitors act on neurons that selectively stimulate energy consumption, thereby exhibiting a therapeutic effect on obesity.

Most existing MAO-B inhibitors are irreversible inhibitors, exhibiting a variety of side effects. Although some progress has been made in the class of reversible MAO-B inhibitors, there is still a need to develop significantly reversible MAO-B inhibitors that can effectively improve cognitive function, treat neurodegenerative diseases, and address obesity.

### SUMMARY

In a first aspect, the present invention provides a compound as shown in formula (I), a tautomer, stereoisomer or pharmaceutically acceptable salt thereof, 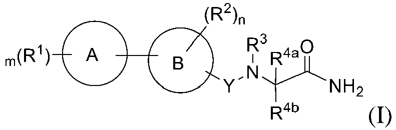 wherein
rings A and B are each independently selected from C₆₋₁₀ aryl and 5- to 10-membered heteroaryl;
R¹ and R² are each independently selected from deuterium, halogen, cyano, nitro, amino, hydroxy, mercapto, -SF₅, -COOH, -OR⁵, -SR⁵, -NR⁵R⁵, -C(O)R⁵, -C(O)OR⁵, -C(O)NR⁵R⁵, - OC(O)R⁵, -S(O)₁₋₂R⁵, -S(O)₂NR⁵R⁵, -N(R⁵)S(O)₂R⁵, -N(R⁵)C(O)R⁵, -N(R⁵)C(O)NR⁵, C₁₋₆ alkyl, C₂₋₆ alkenyl, C₂₋₆ alkynyl, C₃₋₁₀ cycloalkyl, C₃₋₁₀ cycloalkenyl, C₆₋₁₀ aryl, 3- to 10-membered heterocyclyl and 5- to 10-membered heteroaryl; wherein the C₁₋₆ alkyl, C₂₋₆ alkenyl, C₂₋₆ alkynyl, C₃₋₁₀ cycloalkyl, C₃₋₁₀ cycloalkenyl, C₆₋₁₀ aryl, 3- to 10-membered heterocyclyl and 5- to 10-membered heteroaryl are each optionally substituted with 0 to 6 identical or different substituents selected from deuterium, cyano, amino, hydroxyl and halogen;
each R⁵ is independently selected from hydrogen, deuterium, C₁₋₆ alkyl, C₂₋₆ alkenyl, C₂₋₆ alkynyl, C₃₋₁₀ cycloalkyl and 3- to 10-membered heterocyclyl, wherein the C₁₋₆ alkyl, C₂₋₆ alkenyl, C₂₋₆ alkynyl, C₃₋₁₀ cycloalkyl and 3- to 10-membered heterocyclyl are each optionally substituted with 0 to 6 identical or different substituents selected from deuterium, cyano, amino, hydroxyl and halogen;
m and n are each independently selected from 0 to 6;
Y is independently selected from (CR⁶R⁷)₁₋₂ or is a single bond;
R⁶ and R⁷ are each independently selected from hydrogen, deuterium, halogen, C₁₋₆ alkyl, C₁₋₆ haloalkyl, C₁₋₆ alkoxy, C₁₋₆ haloalkoxy, and hydroxy-substituted C₁₋₆ alkyl;
alternatively, when Y is selected from (CR⁶R⁷)₂, one R⁶ or R⁷, together with two carbon atoms, forms C₃₋₆ cycloalkyl or 3- to 6-membered heterocyclyl; the remaining R⁶ and R⁷ are selected from hydrogen, deuterium and halogen; wherein the heterocyclyl contains 0 to 2 heteroatoms selected from N, O, C(=O) and S, and the numbers of the heteroatoms are not 0 simultaneously; the C₃₋₆ cycloalkyl and 3- to 6-membered heterocyclyl are each optionally substituted with 0 to 6 identical or different substituents selected from deuterium, C₁₋₆ alkyl, cyano, amino, hydroxyl and halogen;
alternatively, R² and R⁶, together with the atoms to which they are attached, form C₅₋₈ carbocyclyl or 5- to 8-membered heterocyclyl; wherein the heterocyclyl contains 0 to 2 heteroatoms selected from N, O, C(=O) and S, and the numbers of the heteroatoms are not 0 simultaneously; the C₅₋₈ carbocyclyl and 5- to 8-membered heterocyclyl are each optionally substituted with 0 to 6 identical or different substituents selected from deuterium, cyano, amino, hydroxy, C₁₋₆ alkyl and halogen; R⁷ is selected from hydrogen, deuterium, halogen, C₁₋₆ alkyl, C₁₋₆ haloalkyl, C₁₋₆ alkoxy, C₁₋₆ haloalkoxy and hydroxy-substituted C₁₋₆ alkyl;
R³ is independently selected from hydrogen, deuterium, C₁₋₆ alkyl, C₂₋₆ alkenyl, C₂₋₆ alkynyl, C₁₋₆ haloalkyl, C₁₋₆ alkoxy, C₁₋₆ haloalkoxy, hydroxy-substituted C₁₋₆ alkyl and C₃₋₈ cycloalkyl;
alternatively, R² and R³, together with the atoms to which they are attached, form 4- to 8-membered nitrogen-containing heterocyclyl; wherein the 4- to 8-membered nitrogen-containing heterocyclyl contains 1 to 2 N heteroatoms and 0 to 2 heteroatoms selected from O, C(=O) and S; the 4- to 8-membered nitrogen-containing heterocyclyl is optionally substituted with 0 to 6 identical or different substituents selected from deuterium, C₁₋₆ alkyl, cyano, amino, hydroxyl and halogen;
R^{4a} and R^{4b} are each independently selected from hydrogen, deuterium, halogen, hydroxy, cyano, amino, mercapto, C₁₋₆ alkyl, C₁₋₆ alkoxy, C₂₋₆ alkenyl, C₂₋₆ alkynyl, C₃₋₁₀ cycloalkyl and C_{3- 10} cycloalkenyl; the C₁₋₆ alkyl, C₁₋₆ alkoxy, C₂₋₆ alkenyl, C₂₋₆ alkynyl, C₃₋₁₀ cycloalkyl or C₃₋₁₀ cycloalkenyl is optionally substituted with 0 to 3 substituents selected from deuterium, halogen, hydroxy, amino, nitro, cyano, mercapto, C₁₋₆ alkyl and C₁₋₆ alkoxy;
alternatively, R^{4a} and R^{4b}, together with the carbon atoms to which they are attached, form C₃₋₆ carbocyclyl or 3- to 6-membered heterocyclyl; wherein the heterocyclyl contains 0 to 2 heteroatoms selected from N, O, C(=O) and S, and the numbers of the heteroatoms are not 0 simultaneously; the C₃₋₆ carbocyclyl and 3- to 6-membered heterocyclyl are each optionally substituted with 0 to 6 identical or different substituents selected from deuterium, cyano, amino, hydroxy, halogen and C₁₋₆ alkyl;
alternatively, R³ and R^{4a}, together with the atoms to which they are attached, form 3- to 8-membered nitrogen-containing heterocyclyl; wherein the nitrogen-containing heterocyclyl contains 1 to 3 N heteroatoms and 0 to 2 heteroatoms selected from O, C(=O) and S; the 3- to 8-membered nitrogen-containing heterocyclyl is optionally substituted with 0 to 6 identical or different substituents selected from deuterium, cyano, amino, hydroxy, C₁₋₃ alkyl, C₁₋₃ alkoxy, C₁₋₃ haloalkyl, C₁₋₃ haloalkoxy and halogen; R^{4b} is selected from hydrogen, deuterium, halogen, hydroxy, cyano, amino, mercapto, C₁₋₆ alkyl, C₁₋₆ alkoxy, C₂₋₆ alkenyl, C₂₋₆ alkynyl, C₃₋₁₀ cycloalkyl and C₃₋₁₀ cycloalkenyl; the C₁₋₆ alkyl, C₁₋₆ alkoxy, C₂₋₆ alkenyl, C₂₋₆ alkynyl, C₃₋₁₀ cycloalkyl or C₃₋₁₀ cycloalkenyl is optionally substituted with 0 to 3 substituents selected from deuterium, halogen, hydroxy, amino, nitro, cyano, mercapto, C₁₋₆ alkyl and C₁₋₆ alkoxy.

In some embodiments of the present invention, rings A and B are each independently selected from phenyl, pyridinyl, pyrimidinyl, pyrazinyl, pyridazinyl, thienyl, furyl, pyrrolyl, thiazolyl, oxazolyl, pyrazolyl and imidazolyl;
preferably, rings A and B are each independently selected from phenyl, pyridinyl, thienyl, furyl, pyrrolyl, thiazolyl, oxazolyl, pyrazolyl and imidazolyl;
more preferably, rings A and B are each independently selected from phenyl, pyridinyl, thienyl and thiazolyl;
still more preferably, rings A and B are each independently selected from phenyl.

In some embodiments of the present invention, R¹ and R² are each independently selected from deuterium, halogen, -SF₅, -OR⁵, -SR⁵, -NR⁵R⁵, -C(O)R⁵, -C(O)OR⁵, -C(O)NR⁵R⁵, -OC(O)R⁵, - S(O)₁₋₂R⁵, -S(O)₂NR⁵R⁵, -N(R⁵)S(O)₂R⁵, -N(R⁵)C(O)R⁵, -N(R⁵)C(O)NR⁵, C₁₋₆ alkyl, C₂₋₆ alkenyl, C₂₋₆ alkynyl, C₃₋₁₀ cycloalkyl, C₃₋₁₀ cycloalkenyl, C₆₋₁₀ aryl, 3- to 10-membered heterocyclyl and 5- to 10-membered heteroaryl; wherein the C₁₋₆ alkyl, C₂₋₆ alkenyl, C₂₋₆ alkynyl, C₃₋₁₀ cycloalkyl, C₃₋₁₀ cycloalkenyl, C₆₋₁₀ aryl, 3- to 10-membered heterocyclyl and 5- to 10-membered heteroaryl are each optionally substituted with 0 to 6 identical or different substituents selected from deuterium, cyano, amino, hydroxyl and halogen;
each R⁵ is independently selected from hydrogen, deuterium, C₁₋₆ alkyl, C₂₋₆ alkenyl, C₂₋₆ alkynyl, C₃₋₁₀ cycloalkyl and 3- to 10-membered heterocyclyl; wherein the C₁₋₆ alkyl, C₂₋₆ alkenyl, C₂₋₆ alkynyl, C₃₋₁₀ cycloalkyl and 3- to 10-membered heterocyclyl are each optionally substituted with 0 to 6 identical or different substituents selected from deuterium, cyano, amino, hydroxyl and halogen.

In some preferred embodiments of the present invention, R¹ and R² are each independently selected from deuterium, halogen, -SF₅, -OR⁵, -SR⁵, -NR⁵R⁵, -C(O)R⁵, -C(O)OR⁵, -C(O)NR⁵R⁵, - OC(O)R⁵, -S(O)₁₋₂R⁵, -S(O)₂NR⁵R⁵, -N(R⁵)S(O)₂R⁵, -N(R⁵)C(O)R⁵, -N(R⁵)C(O)NR⁵, C₁₋₆ alkyl, C₃₋₁₀ cycloalkyl and 3- to 10-membered heterocyclyl; wherein the C₁₋₆ alkyl, C₃₋₁₀ cycloalkyl and 3- to 10-membered heterocyclyl are each optionally substituted with 0 to 6 identical or different substituents selected from deuterium, cyano, amino, hydroxyl and halogen;
each R⁵ is independently selected from hydrogen, deuterium, C₁₋₆ alkyl, C₃₋₁₀ cycloalkyl and 3- to 10-membered heterocyclyl, wherein the C₁₋₆ alkyl, C₃₋₁₀ cycloalkyl and 3- to 10- membered heterocyclyl are each optionally substituted with 0 to 6 identical or different substituents selected from deuterium, cyano, amino, hydroxyl and halogen.

In some preferred embodiments of the present invention, R¹ and R² are each independently selected from deuterium, halogen, -SF₅, -OR⁵, -SR⁵, -NR⁵R⁵, -C(O)R⁵, -S(O)₁₋₂R⁵, C₁₋₆ alkyl, C₃₋₁₀ cycloalkyl and 3- to 10-membered heterocyclyl; wherein the C₁₋₆ alkyl, C₃₋₁₀ cycloalkyl and 3-to 10-membered heterocyclyl are each optionally substituted with 0 to 6 identical or different substituents selected from deuterium, cyano, amino, hydroxyl and halogen;
each R⁵ is independently selected from hydrogen, deuterium, C₁₋₆ alkyl, C₃₋₁₀ cycloalkyl and 3- to 10-membered heterocyclyl, wherein the C₁₋₆ alkyl, C₃₋₁₀ cycloalkyl and 3- to 10- membered heterocyclyl are each optionally substituted with 0 to 6 identical or different substituents selected from deuterium, cyano, amino, hydroxyl and halogen.

In some preferred embodiments of the present invention, R¹ and R² are each independently selected from deuterium, halogen, -SF₅, -OR⁵, -SR⁵, C₁₋₆ alkyl, C₃₋₁₀ cycloalkyl and 3- to 10-membered heterocyclyl; wherein the C₁₋₆ alkyl, C₃₋₁₀ cycloalkyl and 3- to 10-membered heterocyclyl are each optionally substituted with 0 to 6 identical or different substituents selected from deuterium, cyano, amino, hydroxyl and halogen;
each R⁵ is independently selected from hydrogen, deuterium, C₁₋₆ alkyl, C₃₋₁₀ cycloalkyl and 3- to 10-membered heterocyclyl, wherein the C₁₋₆ alkyl, C₃₋₁₀ cycloalkyl and 3- to 10- membered heterocyclyl are each optionally substituted with 0 to 6 identical or different substituents selected from deuterium, cyano, amino, hydroxyl and halogen.

In some preferred embodiments of the present invention, R¹ and R² are each independently selected from deuterium, halogen, -SF₅, -OR⁵, -SR⁵, C₁₋₆ alkyl, C₃₋₁₀ cycloalkyl and 3- to 10-membered heterocyclyl; wherein the C₁₋₆ alkyl, C₃₋₁₀ cycloalkyl and 3- to 10-membered heterocyclyl are each optionally substituted with 0 to 6 identical or different substituents selected from deuterium and halogen;
each R⁵ is independently selected from hydrogen, deuterium, C₁₋₆ alkyl, C₃₋₁₀ cycloalkyl and 3- to 10-membered heterocyclyl, wherein the C₁₋₆ alkyl, C₃₋₁₀ cycloalkyl and 3- to 10- membered heterocyclyl are each optionally substituted with 0 to 6 identical or different substituents selected from deuterium and halogen.

In some preferred embodiments of the present invention, R¹ and R² are each independently selected from deuterium, halogen, -SF₅, -OR⁵, -SR⁵, C₁₋₆ alkyl, C₃₋₆ cycloalkyl and 3- to 6-membered heterocyclyl; wherein the C₁₋₆ alkyl, C₃₋₆ cycloalkyl and 3- to 6-membered heterocyclyl are each optionally substituted with 0 to 6 identical or different substituents selected from deuterium and halogen;
each R⁵ is independently selected from hydrogen, deuterium, C₁₋₆ alkyl, C₃₋₆ cycloalkyl and 3-to 6-membered heterocyclyl, wherein the C₁₋₆ alkyl, C₃₋₆ cycloalkyl and 3- to 6- membered heterocyclyl are each optionally substituted with 0 to 6 identical or different substituents selected from deuterium and halogen.

In some preferred embodiments of the present invention, R¹ and R² are each independently selected from deuterium, fluorine, chlorine, bromine, -SF₅, -OR⁵, -SR⁵, C₁₋₆ alkyl, C₃₋₆ cycloalkyl and 3- to 6-membered heterocyclyl; wherein the C₁₋₆ alkyl, C₃₋₆ cycloalkyl and 3- to 6-membered heterocyclyl are each optionally substituted with 0 to 6 identical or different substituents selected from deuterium, fluorine, chlorine and bromine;
each R⁵ is independently selected from C₁₋₆ alkyl, C₃₋₆ cycloalkyl and 3- to 6-membered heterocyclyl, wherein the C₁₋₆ alkyl, C₃₋₆ cycloalkyl and 3- to 6- membered heterocyclyl are each optionally substituted with 0 to 6 identical or different substituents selected from deuterium, fluorine, chlorine and bromine.

In some preferred embodiments of the present invention, R¹ and R² are each independently selected from fluorine, chlorine, -SF₅, -SCF₃, methyl, ethyl, propyl, isopropyl, cyclopropyl, butyl, isobutyl, tert-butyl, cyclobutyl, methoxy, ethoxy, propoxy, isopropoxy, cyclopropoxy, butoxy, isobutoxy, tert-butoxy and cyclobutoxy; wherein methyl, ethyl, propyl, isopropyl, cyclopropyl, butyl, isobutyl, tert-butyl, cyclobutyl, methoxy, ethoxy, propoxy, isopropoxy, cyclopropoxy, butoxy, isobutoxy, tert-butoxy and cyclobutoxy are each optionally substituted with 0 to 6 identical or different substituents selected from deuterium, fluorine, chlorine and bromine.

In some preferred embodiments of the present invention, R¹ and R² are each independently selected from fluorine, chlorine, -SF₅, -SCF₃, -OCF₃, -CF₃, methyl, deuterated methyl and cyclopropyl.

In some preferred embodiments of the present invention, R¹ is selected from -CF₃, and R² is selected from fluorine.

In some embodiments of the present invention, m and n are each independently selected from 0 to 5, preferably 0, 1, 2, 3, 4, and 5, and more preferably 0, 1, 2 and 3.

In some embodiments of the present invention, m is selected from 0 to 3, preferably 1, 2 and 3, more preferably 1; n is selected from 0 and 1.

In some embodiments of the present invention, Y is independently selected from (CR⁶R⁷)₁₋₂;
R⁶ and R⁷ are each independently selected from hydrogen, deuterium, halogen, C₁₋₆ alkyl, C₁₋₆ haloalkyl, C₁₋₆ alkoxy, C₁₋₆ haloalkoxy, and hydroxy-substituted C₁₋₆ alkyl;
alternatively, when Y is selected from (CR⁶R⁷)₂, one R⁶ or R⁷, together with two carbon atoms, forms C₃₋₆ cycloalkyl or 3- to 6-membered heterocyclyl; the remaining R⁶ and R⁷ are selected from hydrogen, deuterium and halogen; wherein the heterocyclyl contains 0 to 2 heteroatoms selected from N, O, C(=O) and S, and the numbers of the heteroatoms are not 0 simultaneously; the C₃₋₆ cycloalkyl and 3- to 6-membered heterocyclyl are each optionally substituted with 0 to 6 identical or different substituents selected from deuterium, cyano, amino, hydroxyl and halogen;
alternatively, R² and R⁶, together with the atoms to which they are attached, form C₅₋₈ carbocyclyl or 5- to 8-membered heterocyclyl; wherein the heterocyclyl contains 0 to 2 heteroatoms selected from N, O, C(=O) and S, and the numbers of the heteroatoms are not 0 simultaneously; the C₅₋₈ carbocyclyl and 5- to 8-membered heterocyclyl are each optionally substituted with 0 to 6 identical or different substituents selected from deuterium, cyano, amino, hydroxy, and halogen; R⁷ is selected from hydrogen, deuterium, halogen, C₁₋₆ alkyl, C₁₋₆ haloalkyl, C₁₋₆ alkoxy, C₁₋₆ haloalkoxy and hydroxy-substituted C₁₋₆ alkyl.

In some preferred embodiments of the present invention, Y is independently selected from (CR⁶R⁷)₁₋₂;
R⁶ and R⁷ are each independently selected from hydrogen, deuterium, methyl and halogen;
alternatively, R⁶ and R⁷, together with the carbon atoms to which they are attached, form 3-membered cycloalkyl; wherein the 3-membered cycloalkyl is optionally substituted with 0 to 4 identical or different substituents selected from deuterium, cyano, amino, hydroxyl and halogen;
alternatively, R² and R⁶, together with the atoms to which they are attached, form C₅₋₆ carbocyclyl or 5- to 6-membered heterocyclyl; wherein the heterocyclyl contains 0 to 2 heteroatoms selected from N, O, C(=O) and S, and the numbers of the heteroatoms are not 0 simultaneously; the C₅₋₆ carbocyclyl and 5- to 6-membered heterocyclyl are each optionally substituted with 0 to 6 identical or different substituents selected from deuterium and halogen; R⁷ is selected from hydrogen, deuterium, methyl and halogen.

In some preferred embodiments of the present invention, Y is independently selected from (CR⁶R⁷)₁₋₂;
R⁶ and R⁷ are each independently selected from hydrogen and methyl;
alternatively, when Y is selected from (CR⁶R⁷)₂, one R⁶ or R⁷, together with two carbon atoms, forms 3-membered cycloalkyl;
alternatively, R² and R⁶, together with the atoms to which they are attached, form 5-membered carbocyclyl; R⁷ is selected from hydrogen and methyl.

In some embodiments of the present invention, R³ is independently selected from hydrogen, deuterium, C₁₋₆ alkyl, C₂₋₆ alkynyl and C₃₋₈ cycloalkyl;
alternatively, R² and R³, together with the atoms to which they are attached, form 5- to 6-membered nitrogen-containing heterocyclyl, wherein the 5- to 6-membered nitrogen-containing heterocyclyl is optionally substituted with 0 to 3 identical or different substituents selected from deuterium, halogen and C₁₋₆ alkyl.

In some preferred embodiments of the present invention, R³ is independently selected from hydrogen and propynyl;
alternatively, R² and R³, together with the atoms to which they are attached, form 5- to 6-membered nitrogen-containing heterocyclyl, wherein the 5- to 6-membered nitrogen-containing heterocyclyl is optionally substituted with 0 to 3 identical or different substituents selected from C₁₋₆ alkyl.

In some embodiments of the present invention, R^{4a} and R^{4b} are each independently selected from hydrogen, halogen and C₁₋₆ alkyl, wherein the C₁₋₆ alkyl is optionally substituted with 0 to 3 identical or different substituents selected from deuterium, amino, hydroxy, C₁₋₃ alkyl, C₁₋₃ alkoxy, C₁₋₃ haloalkyl, C₁₋₃ haloalkoxy and halogen;
alternatively, R^{4a} and R^{4b}, together with the carbon atoms to which they are attached, form C₃₋₄ cycloalkyl or 3- to 4-membered heterocycloalkyl; wherein the heterocycloalkyl contains 1 heteroatom selected from O, N, C(=O) and S; the C₃₋₄ cycloalkyl and 3- to 4-membered heterocycloalkyl are each optionally substituted with 0 to 6 identical or different substituents selected from deuterium and halogen;
alternatively, R³ and R^{4a}, together with the atoms to which they are attached, form 3- to 7-membered nitrogen-containing heterocyclyl; wherein the nitrogen-containing heterocyclyl contains 1 to 3 N atoms and 0 to 3 heteroatoms selected from O and C(=O); wherein the 3- to 7-membered heterocyclyl is optionally substituted with 0 to 6 identical or different substituents selected from deuterium, amino, hydroxy, C₁₋₃ alkyl, C₁₋₃ alkoxy, C₁₋₃ haloalkyl, C₁₋₃ haloalkoxy and halogen; R^{4b} is selected from hydrogen, halogen and C₁₋₆ alkyl, wherein the C₁₋₆ alkyl is optionally substituted with 0 to 3 identical or different substituents selected from deuterium, amino, hydroxy, C₁₋₃ alkyl, C₁₋₃ alkoxy, C₁₋₃ haloalkyl, C₁₋₃ haloalkoxy and halogen.

In some preferred embodiments of the present invention, R^{4a} and R^{4b} are each independently selected from hydrogen, fluorine and methyl;
alternatively, R^{4a} and R^{4b}, together with the carbon atoms to which they are attached, form C₃₋₄ cycloalkyl or 3- to 4-membered heterocycloalkyl; wherein the heterocycloalkyl contains 1 O atom; the C₃₋₄ cycloalkyl and 3- to 4-membered heterocycloalkyl are each optionally substituted with 0 to 6 identical or different halogens;
alternatively, R³ and R^{4a}, together with the atoms to which they are attached, form 3- to 7-membered nitrogen-containing heterocyclyl; wherein the nitrogen-containing heterocyclyl contains 1 to 3 N atoms, and 0 to 3 heteroatoms selected from O and C(=O); the 3- to 7-membered nitrogen-containing heterocyclyl is optionally substituted with 0 to 6 identical or different substituents selected from amino, hydroxy, C₁₋₃ alkyl and halogen;
alternatively, R³ and R^{4a}, together with the atoms to which they are attached, form 3- to 6-membered nitrogen-containing heterocyclyl; wherein the nitrogen-containing heterocyclyl contains 1 N atom; the 3- to 6-membered nitrogen-containing heterocyclyl is optionally substituted with 0 to 3 identical or different substituents selected from amino, hydroxy, C₁₋₃ alkyl and halogen;
alternatively, R³ and R^{4a}, together with the atoms to which they are attached, form 3- to 5-membered nitrogen-containing heterocyclyl; wherein the nitrogen-containing heterocyclyl contains 1 N atom; the 3- to 5-membered nitrogen-containing heterocyclyl is optionally substituted with 0 to 2 identical or different substituents selected from amino, hydroxy, C₁₋₃ alkyl and halogen;
alternatively, R³ and R^{4a}, together with the atoms to which they are attached, form 4-membered nitrogen-containing heterocyclyl; wherein the nitrogen-containing heterocyclyl contains 1 N atom; the 4-membered nitrogen-containing heterocyclyl is optionally substituted with 0 to 2 identical or different substituents selected from amino, hydroxy, C₁₋₃ alkyl and halogen.

In some preferred embodiments of the present invention, R^{4a} and R^{4b} are each independently selected from hydrogen, fluorine and methyl;
alternatively, R^{4a} and R^{4b}, together with the carbon atoms to which they are attached, form cyclopropyl, cyclobutyl or a 4-membered oxacycle; wherein the cyclopropyl, cyclobutyl and 4-membered oxacycle are each optionally substituted with 0 to 6 fluorines;
alternatively, R³ and R^{4a}, together with the atoms to which they are attached, form nitrogen-containing 3-membered monocyclic heterocyclyl, 4-membered monocyclic heterocyclyl, 5-membered monocyclic heterocyclyl, 6-membered monocyclic heterocyclyl, 7-membered monocyclic heterocyclyl, 5-membered spiro heterocyclyl, 5-membered bridged heterocyclyl, 6-membered spiro heterocyclyl, 6-membered bridged heterocyclyl, 6-membered fused heterocyclyl, 7-membered spiro heterocyclyl, 7-membered bridged heterocyclyl or 7-membered fused heterocyclyl; wherein the nitrogen-containing heterocyclyl contains 1 to 3 N atoms and 0 to 3 heteroatoms selected from O and C(=O); the nitrogen-containing heterocyclyl is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 3-membered monocyclic heterocyclyl, 4-membered monocyclic heterocyclyl, 5-membered monocyclic heterocyclyl, 6-membered monocyclic heterocyclyl or 7-membered monocyclic heterocyclyl; wherein the nitrogen-containing heterocyclyl contains 1 to 3 N atoms and 0 to 3 heteroatoms selected from O and C(=O); the nitrogen-containing heterocyclyl is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 3-membered monocyclic heterocyclyl, 4-membered monocyclic heterocyclyl, 5-membered monocyclic heterocyclyl, or 6-membered monocyclic heterocyclyl; wherein the nitrogen-containing heterocyclyl contains 1 to 3 N atoms and 0 to 3 heteroatoms selected from O and C(=O); the nitrogen-containing heterocyclyl is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 3-membered monocyclic heterocyclyl, 4-membered monocyclic heterocyclyl, 5-membered monocyclic heterocyclyl, or 6-membered monocyclic heterocyclyl; wherein the nitrogen-containing heterocyclyl contains 1 N atom; the nitrogen-containing heterocyclyl is optionally substituted with 0 to 3 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 3-membered monocyclic heterocyclyl, 4-membered monocyclic heterocyclyl, or 5-membered monocyclic heterocyclyl; wherein the nitrogen-containing heterocyclyl contains 1 N atom; the nitrogen-containing heterocyclyl is optionally substituted with 0 to 2 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 4-membered monocyclic heterocyclyl; wherein the nitrogen-containing heterocyclyl contains 1 N atom; the nitrogen-containing heterocyclyl is optionally substituted with 0 to 2 identical or different substituents selected from amino, hydroxy, methyl, and fluorine; R^{4b} is selected from hydrogen, fluorine and methyl.

In some preferred embodiments of the present invention, R^{4a} and R^{4b} are each independently selected from hydrogen, fluorine and methyl;
alternatively, R^{4a} and R^{4b}, together with the carbon atoms to which they are attached, form cyclopropyl, cyclobutyl or a 4-membered oxacycle; wherein the cyclopropyl, cyclobutyl and 4-membered oxacycle are each optionally substituted with 0 to 6 fluorines;
alternatively, R³ and R^{4a}, together with the atoms to which they are attached, form a nitrogen-containing heterocyclyl group selected from 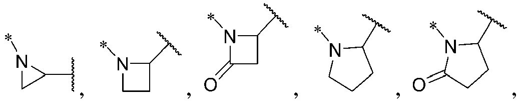 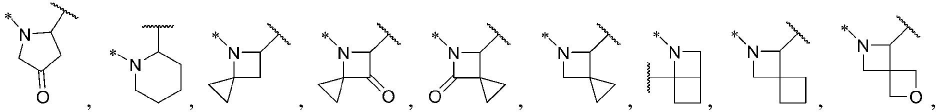 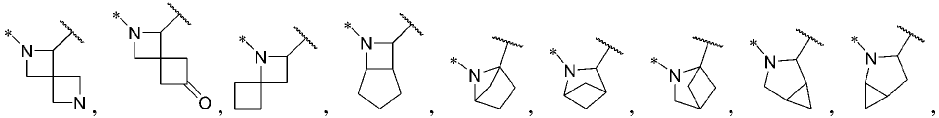 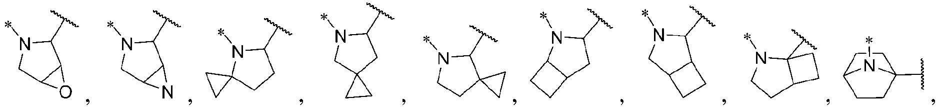 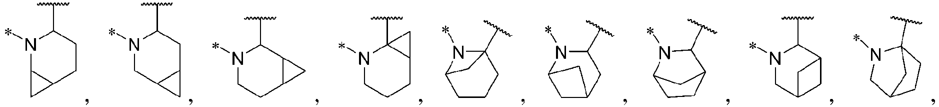 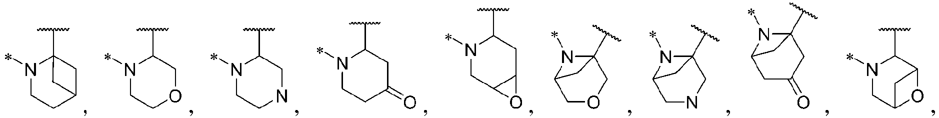 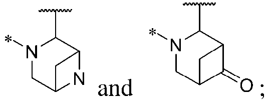 wherein 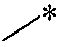 denotes the linkage to the Y end and 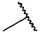 denotes the linkage to C(O)NH₂; each of the nitrogen-containing heterocyclyl groups is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl.

In some preferred embodiments of the present invention, R^{4a} and R^{4b} are each independently selected from hydrogen, fluorine and methyl;
alternatively, R^{4a} and R^{4b}, together with the carbon atoms to which they are attached, form cyclopropyl, cyclobutyl or a 4-membered oxacycle; wherein the cyclopropyl, cyclobutyl and 4-membered oxacycle are each optionally substituted with 0 to 6 fluorines;
alternatively, R³ and R^{4a}, together with the atoms to which they are attached, form a nitrogen-containing heterocyclyl group selected from 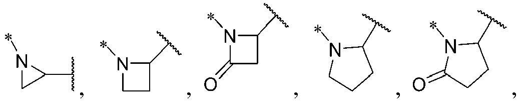 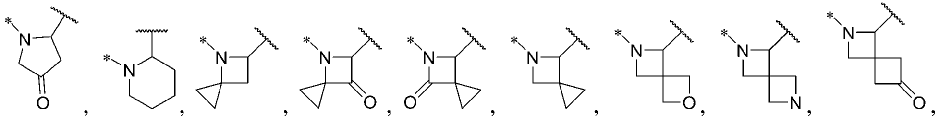 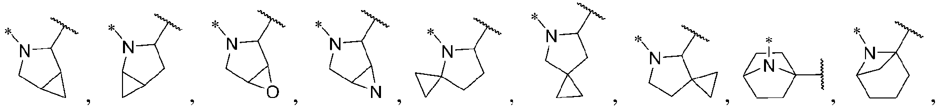 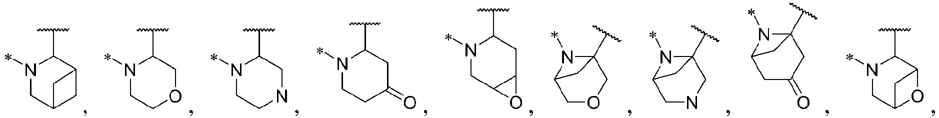 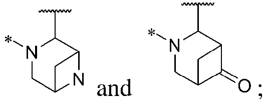 wherein 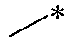 denotes the linkage to the Y end and 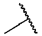 denotes the linkage to the C(O)NH₂ end; each of the nitrogen-containing heterocyclyl groups is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl.

In some preferred embodiments of the present invention, R^{4a} and R^{4b} are each independently selected from hydrogen, fluorine and methyl;
alternatively, R^{4a} and R^{4b}, together with the carbon atoms to which they are attached, form cyclopropyl, cyclobutyl or a 4-membered oxacycle; wherein the cyclopropyl, cyclobutyl and 4-membered oxacycle are each optionally substituted with 0 to 6 fluorines;
alternatively, R³ and R^{4a}, together with the atoms to which they are attached, form 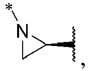 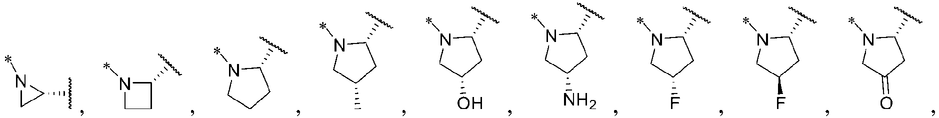 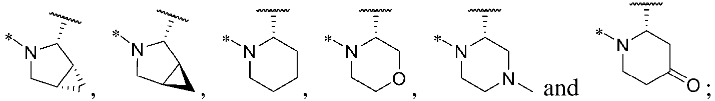 wherein 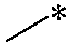 denotes the linkage to the Y end and 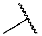 denotes the linkage to the C(O)NH₂ end; R^{4b} is selected from hydrogen, fluorine and methyl;
alternatively, R³ and R^{4a}, together with the atoms to which they are attached, form 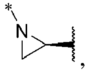 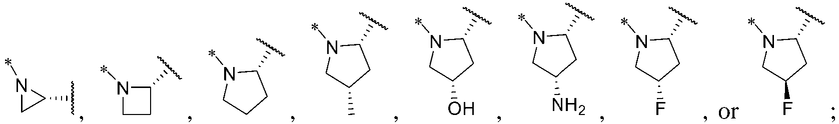 wherein 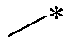 denotes the linkage to the Y end and 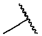 denotes the linkage to the C(O)NH₂ end; R^{4b} is selected from hydrogen, fluorine and methyl;
alternatively, R³ and R^{4a}, together with the atoms to which they are attached, form 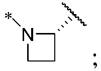 wherein 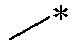 denotes the linkage to the Y end and 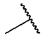 denotes the linkage to the C(O)NH₂ end; R^{4b} is selected from hydrogen, fluorine and methyl;
alternatively, R³ and R^{4a}, together with the atoms to which they are attached, form 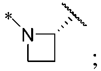 wherein 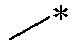 denotes the linkage to the Y end and 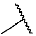 denotes the linkage to the C(O)NH₂ end; R^{4b} is selected from hydrogen.

In the above embodiments of the present invention, except for the groups defined above, all other groups are as defined herein, and the descriptions thereof are omitted for brevity.

The above embodiments of the present invention may be combined in any combination, and any resulting combined embodiments are also encompassed within the scope of the embodiments of the present invention.

In the above technical solutions, the compound of formula (I) does not include the following compounds:

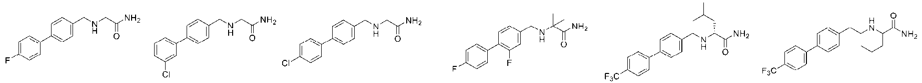

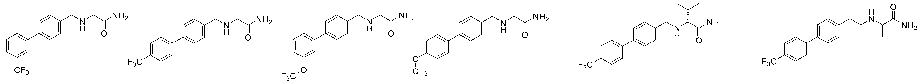

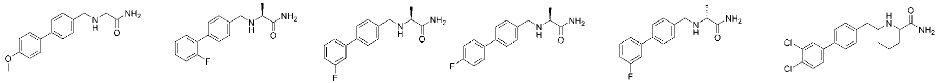

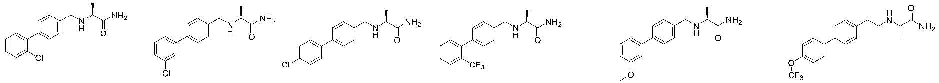

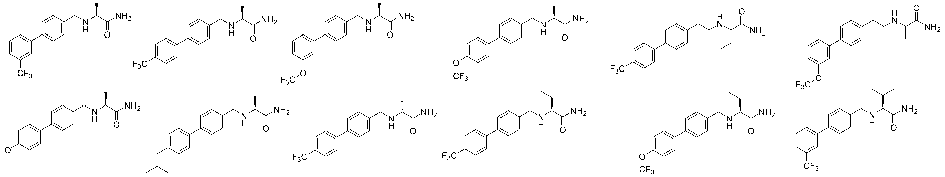

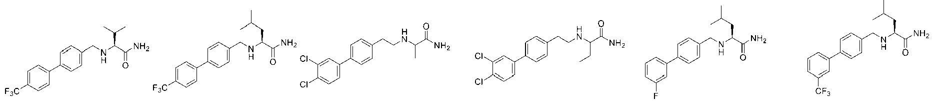

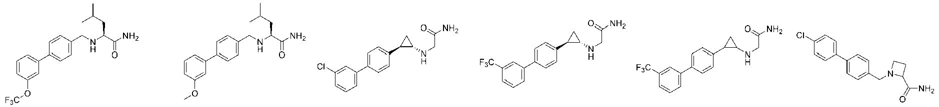

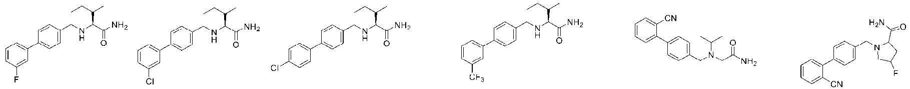

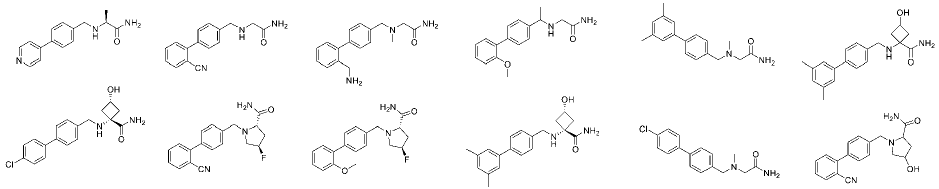

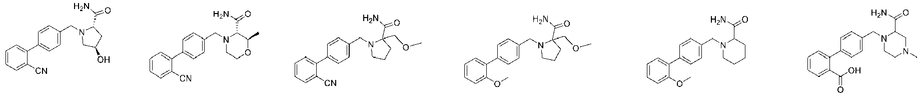

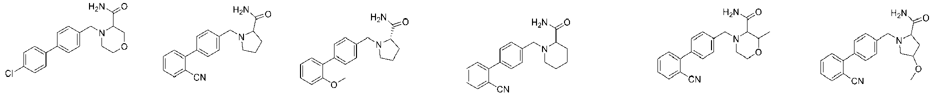

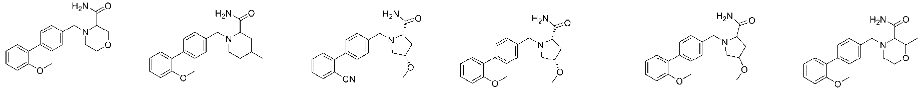

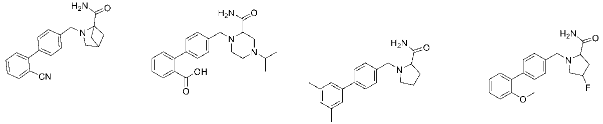

Exemplary structures are set forth below, including but not limited to compounds having the structural formulas shown below, tautomers, stereoisomers, or pharmaceutically acceptable salts thereof:

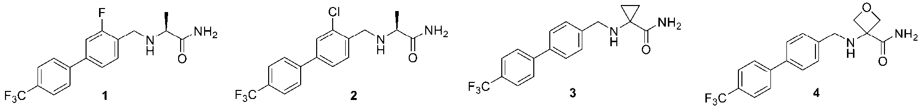

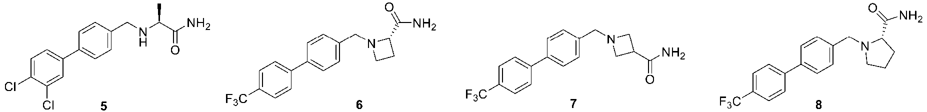

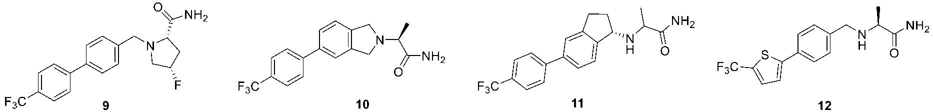

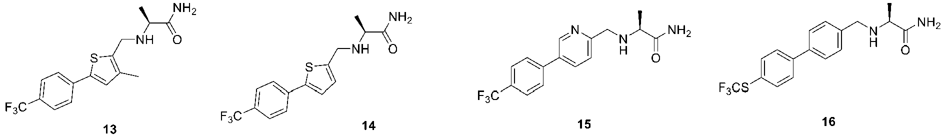

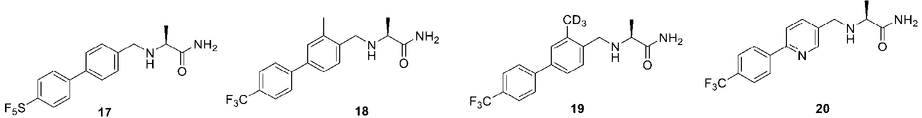

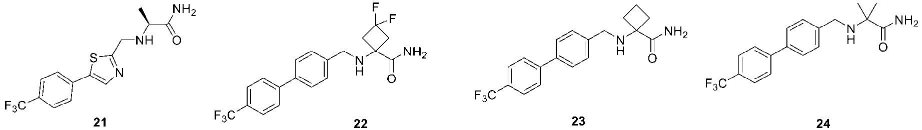

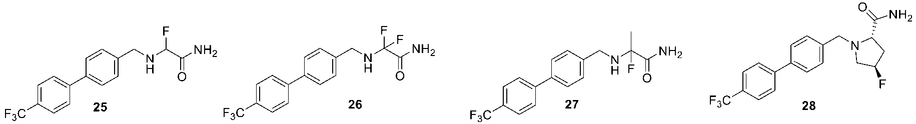

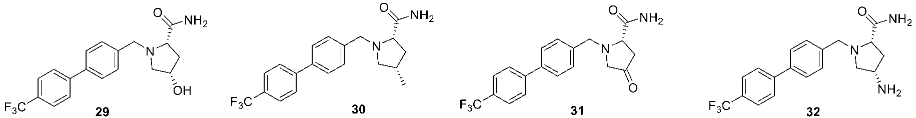

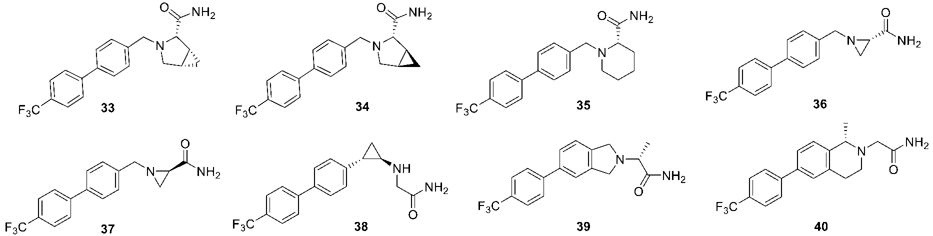

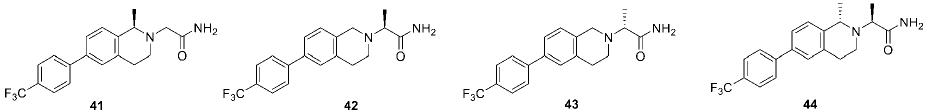

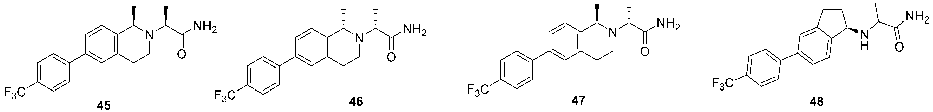

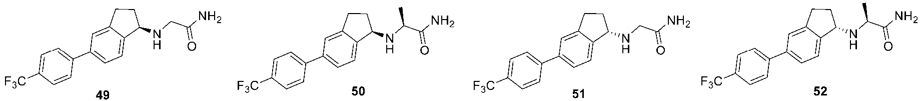

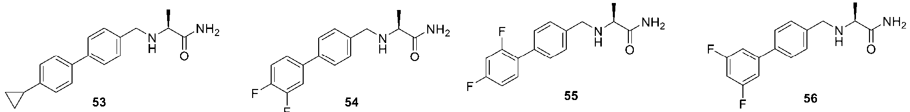

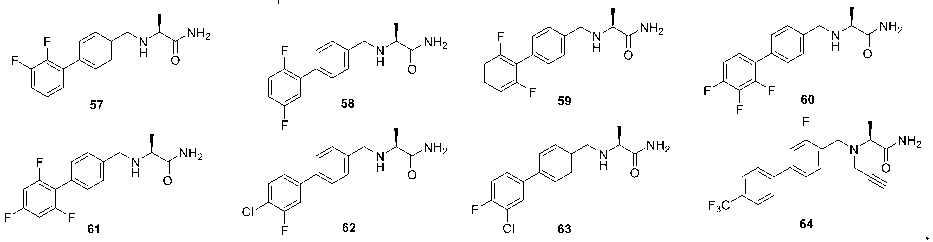

In some preferred embodiments of the present invention, the pharmaceutically acceptable salt is selected from: methanesulfonate salts.

In a second aspect, the present invention provides a pharmaceutical composition comprising the aforementioned compound, a tautomer, stereoisomer or pharmaceutically acceptable salt thereof, and optionally further comprising a pharmaceutically acceptable carrier.

In a third aspect, the present invention provides use of the aforementioned compound, a tautomer, stereoisomer or pharmaceutically acceptable salt thereof, or the pharmaceutical composition according to the second aspect of the present invention in the preparation of a medicament for diseases or disorders associated with overexpression of monoamine oxidase-B (MAO-B).

In some embodiments of the present invention, the medicament is used for preventing, ameliorating and/or treating diseases or disorders related to memory, cognition, mood, neurodegeneration, obesity and the like.

In some embodiments of the present invention, the disease or disorder is selected from Alzheimer's disease, depression, stroke, Parkinson's disease, hypertension and obesity.

In a fourth aspect, the present invention provides a method for preventing, ameliorating and/or treating diseases or disorders associated with overexpression of monoamine oxidase-B (MAO-B), comprising administering to a patient a therapeutically effective amount of the aforementioned compound, a tautomer, stereoisomer or pharmaceutically acceptable salt thereof, or the pharmaceutical composition described herein; preferably, the diseases or disorders associated with overexpression of monoamine oxidase-B (MAO-B) are those related to memory, cognition, mood, neurodegeneration, obesity and the like, preferably: Alzheimer's disease, depression, stroke, Parkinson's disease, hypertension or obesity, etc.

In a fifth aspect, the present invention provides a medicament comprising the aforementioned compound, a tautomer, stereoisomer or pharmaceutically acceptable salt thereof, wherein the medicament is used for preventing, ameliorating and/or treating diseases or disorders associated with overexpression of monoamine oxidase-B (MAO-B); the diseases or disorders associated with overexpression of monoamine oxidase-B (MAO-B) are those related to memory, cognition, mood, neurodegeneration, obesity and the like, preferably: Alzheimer's disease, depression, stroke, Parkinson's disease, hypertension or obesity, etc.

In a sixth aspect, the present invention provides a method for preparing a compound as shown in formula (I), a tautomer, stereoisomer or pharmaceutically acceptable salt thereof, wherein the compound can be prepared via the steps set forth below: 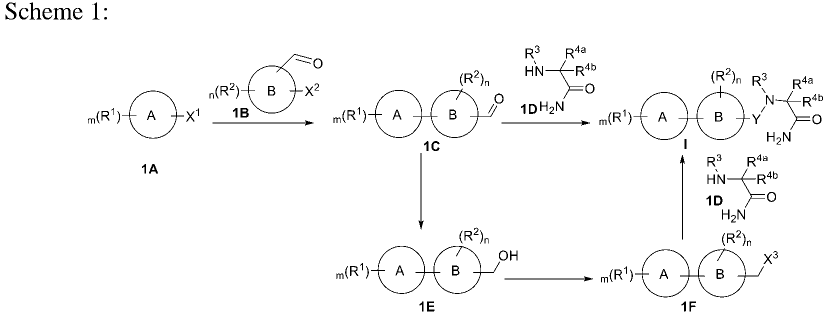
wherein: ring A, ring B, R¹, R², R³, R^{4a}, R^{4b}, m and n are defined herein;
X¹, X² and X³ each represent a leaving group, which is preferably selected from halogen, OMs, OTf, OTs, B(OH)₂, Bpin and Bneop.

Further preferably: when X¹ is Cl, Br, I, OMs, OTf, or OTs, X² is B(OH)₂, Bpin or Bneop;
alternatively, when X¹ is B(OH)₂, Bpin or Bneop, X² is Cl, Br, I, OMs, OTf, or OTs;
X³ is Cl, Br, I, OMs, OTf, or OTs;
wherein
   (1) 1A and 1B react in the presence of a suitable catalyst to afford 1C;
   (2) 1C undergoes reductive amination with 1D or a salt thereof to yield the compound as shown in formula (I);
      or alternatively,
   (3) 1C is converted into 1E under reductive conditions;
   (4) 1E is converted into 1F with a leaving group;
   (5) 1F reacts with 1D under basic conditions to give the compound as shown in formula (I);
wherein:
the catalyst mentioned in step (1) is preferably a transition metal catalyst, and more preferably palladium, nickel, etc.;
the reducing agent used in steps (2) and (3) is preferably sodium borohydride, sodium cyanoborohydride, etc.;
the base used in step (5) is an organic or inorganic base, such as: lithium carbonate, sodium carbonate, potassium carbonate, cesium carbonate, sodium bicarbonate, potassium bicarbonate, sodium phosphate, potassium phosphate, triethylamine, N,N-diisopropylethylamine or 1,8-diazabicyclo[5.4.0]undec-7-ene;
all above reactions can be carried out in a suitable solvent conventionally used in the art; preferably, the solvent is one solvent or a mixed solvent of two or more selected from benzene, toluene, methanol, ethanol, isopropanol, water, N,N-dimethylformamide, dimethyl sulfoxide, acetone, ethyl acetate, tetrahydrofuran, 1,4-dioxane, dichloromethane, chloroform, 1,2-dichloroethane, N-methylpyrrolidone and N,N-dimethylacetamide;
the above reaction can be carried out at a suitable temperature, preferably, the reaction temperature is -20 °C to 150 °C, and more preferably -10 °C to 120 °C.

Intermediates or target products obtained from the reactions can be purified by conventional purification methods well known in the art as needed, such as column chromatography, thin-layer chromatography and the like. 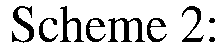 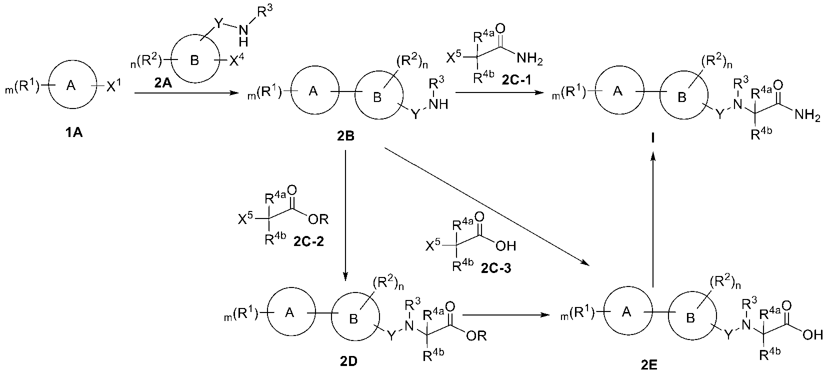
wherein: ring A, ring B, R¹, R², R³, R^{4a}, R^{4b}, R, Y, m and n are defined herein;
X¹, X⁴ and X⁵ each represent a leaving group, which is preferably selected from halogen, OMs, OTf, OTs, B(OH)₂, Bpin and Bneop.

Further preferably: when X¹ is Cl, Br, I, OMs, OTf, or OTs, X⁴ is B(OH)₂, Bpin or Bneop;
alternatively, when X¹ is B(OH)₂, Bpin or Bneop, X⁴ is Cl, Br, I, OMs, OTf, or OTs;
X⁵ is Cl, Br, I, OMs, OTf, or OTs;
wherein
   (1) 1A and 2A react in the presence of a suitable catalyst to afford 2B;
   (2) 2B reacts with 2C-1 under basic conditions to give the compound as shown in formula (I); alternatively,
   (3) 2B and 2C-2 react under basic conditions to give 2D;
   (4) 2D reacts to give 2E under basic conditions;
      alternatively,
   (5) 2B and 2C-3 react under basic conditions to give 2E;
   (6) 2E undergoes a condensation reaction with ammonia under basic conditions to afford the compound as shown in formula (I);
wherein:
the catalyst mentioned in step (1) is preferably a transition metal catalyst, and more preferably palladium, nickel, etc.;
the base used in steps (2), (3), (5) and (6) is an organic or inorganic base, such as: lithium carbonate, sodium carbonate, potassium carbonate, cesium carbonate, sodium bicarbonate, potassium bicarbonate, sodium phosphate, potassium phosphate, triethylamine, N,N-diisopropylethylamine or 1,8-diazabicyclo[5.4.0]undec-7-ene;
the base used in step (4) is lithium hydroxide, sodium hydroxide or potassium hydroxide.

The ammonia mentioned in step (6) is selected from NH₃ and ammonium salts, such as ammonium chloride, ammonium nitrate, ammonium bicarbonate, ammonium carbonate, ammonium sulfate, and ammonium bisulfate.

All above reactions can be carried out in a suitable solvent conventionally used in the art; preferably, the solvent is one solvent or a mixed solvent of two or more selected from benzene, toluene, methanol, ethanol, isopropanol, water, N,N-dimethylformamide, dimethyl sulfoxide, acetone, ethyl acetate, tetrahydrofuran, 1,4-dioxane, dichloromethane, chloroform, 1,2-dichloroethane, N-methylpyrrolidone and N,N-dimethylacetamide;
the above reaction can be carried out at a suitable temperature, preferably, the reaction temperature is -20 °C to 150 °C, and more preferably -10 °C to 120 °C.

Intermediates or target products obtained from the reactions can be purified by conventional purification methods well known in the art as needed, such as column chromatography, thin-layer chromatography and the like.

### Definitions

Unless otherwise specified, D in the present invention represents deuterium (²H).

Unless otherwise specified, the term "pharmaceutically acceptable salt" or "medicinally acceptable salt" means a salt that, within the limits of reasonable medical judgment, is suitable for contact with the tissues of mammals, particularly humans, without excessive toxicity, irritation, allergic reactions, etc., and is commensurate with a reasonable benefit/risk ratio. For example, pharmaceutically acceptable salts of amines, carboxylic acids, and other types of compounds are well known in the art. The salt can be prepared in situ during the final separation and purification of the compounds of the present invention, or it can be prepared alone by reacting a free base or free acid with a suitable reagent.

Except as otherwise specified, the pharmaceutically acceptable salts of the compounds of the present invention further include their "solvates". The term "solvate" means the physical association of the salt of the compounds of the present invention with one or more solvent molecules (whether organic or inorganic). This physical association includes hydrogen bonds. In certain situations, such as when one or more solvent molecules are incorporated into the lattice of a crystalline solid, the solvate can be separated. Solvent molecules in a solvate can exist in a regular or disordered arrangement. Solvates may contain stoichiometric or non-stoichiometric solvent molecules. "Solvates" encompass both solution phases and separable solvates. Exemplary solvates include, but are not limited to, hydrates, ethanolates, methanolates and isopropanolates. Methods for solvation are well known in the art.

Except as otherwise specified, the pharmaceutically acceptable salts of the compounds of the present invention also include their "hydrates". The term "hydrate" refers to a substance formed when water molecules bind to cations or anions of a compound via coordinate bonds or covalent bonds, or when water molecules occupy defined sites within the solid crystal lattice at a fixed stoichiometric ratio without direct bonding to the cations or anions.

Except as otherwise specified, the compounds of the present invention further encompass their "prodrugs". A prodrug is a pharmaceutical agent that converts into the parent drug in vivo. Prodrugs are frequently advantageous for ameliorating certain undesirable physical or biological properties. Physical properties generally relate to solubility (excess or insufficient lipophilicity or aqueous solubility) or chemical stability, whereas problematic biological characteristics include overly rapid metabolic clearance or poor bioavailability, which may itself correlate with physicochemical attributes. For instance, certain prodrugs are orally bioavailable while their corresponding parent drugs are not. Prodrugs may also exhibit improved solubility in pharmaceutical compositions relative to parent drugs. By way of non-limiting example, the compound of the present invention may be administered in ester form ("prodrug") to facilitate transmembrane transport: where high aqueous solubility hinders membrane permeation, yet such aqueous solubility becomes favorable once the compound enters intracellular compartments; the prodrug is subsequently metabolized and hydrolyzed to yield the carboxylic acid, the active entity. Another example of a prodrug can be a short peptide (polyamino acid) conjugated to an acid group, in which the peptide is metabolized to reveal the active moiety.

Except as otherwise specified, the compounds of the present invention further include their "solvates". The term "solvate" means the physical association of the compounds of the present invention with one or more solvent molecules (whether organic or inorganic). This physical association includes hydrogen bonds. In certain situations, such as when one or more solvent molecules are incorporated into the lattice of a crystalline solid, the solvate can be separated. Solvent molecules in a solvate can exist in a regular or disordered arrangement. Solvates may contain stoichiometric or non-stoichiometric solvent molecules. "Solvates" encompass both solution phases and separable solvates. Exemplary solvates include, but are not limited to, hydrates, ethanolates, methanolates and isopropanolates. Methods for solvation are well known in the art.

Except as otherwise specified, the compounds of the present invention also include their "hydrates". The term "hydrate" refers to a substance formed when water molecules bind to cations or anions of a compound via coordinate bonds or covalent bonds, or when water molecules occupy defined sites within the solid crystal lattice at a fixed stoichiometric ratio without direct bonding to the cations or anions.

Unless otherwise specified, the term "stereoisomer" refers to compounds that have the same chemical structure but whose atoms or groups are arranged differently in space. Stereoisomers include enantiomers, diastereomers, conformational isomers (rotational isomers), geometric isomers (cis/trans) isomers, and atropisomers. Any mixture of stereoisomers obtained can be separated into pure or substantially pure geometric isomers, enantiomers, and diastereomers based on differences in the physicochemical properties of the components, for example, by chromatography and/or fractional crystallization.

Except as otherwise specified, the term "geometric isomers (cis/trans isomers)" encompasses carbon-carbon or carbon-nitrogen double bonds bearing E or Z configurations, wherein the designation "E" indicates higher-priority substituents positioned on opposite sides of the C=C or C=N double bond, and the designation "Z" indicates higher-priority substituents located on the same side of the C=C or C=N double bond (priority is determined per the Cahn-Ingold-Prelog priority rules). The compounds of the present invention may also exist as mixtures of "E" and "Z" isomers.

Except as otherwise specified, the term "tautomers" refers to structural isomers possessing differing energies that are interconvertible via a low energy barrier. Where tautomerization is feasible (e.g., in solution), chemical equilibrium between tautomers can be established. For example, prototropic tautomers (also termed proton-shift tautomers) undergo interconversion through proton migration, such as keto-enol tautomerization and imine-enamine tautomerization. Valence tautomers interconvert via rearrangement of certain bonding electrons.

Unless otherwise indicated, the structural formulas described in the present invention include all isomeric forms (such as enantiomers, diastereomers, and geometric isomers (or conformational isomers)): for example, R and S configurations containing asymmetric centers, (Z) and (E) isomers for double bonds, and (Z) and (E) conformational isomers. Therefore, individual stereochemical isomers or mixtures of enantiomers, diastereomers or geometric (or conformational) isomers of the inventive compounds all fall within the scope of the present invention.

Except as otherwise specified, the term "carbocyclyl" refers to a non-aromatic cyclic hydrocarbon group having 3 to 14 ring carbon atoms ("C₃₋₁₄ carbocyclyl") that contains no heteroatoms within the non-aromatic ring system. In some embodiments, the carbocyclyl group has 3 to 8 ring carbon atoms ("C₃₋₈ carbocyclyl"), 3 to 6 ring carbon atoms ("C₃₋₆ carbocyclyl"), or 5 to 8 ring carbon atoms ("C₅₋₈ carbocyclyl"). In some embodiments, the carbocyclyl group contains 5 to 6 ring carbon atoms ("C₅-₆ carbocyclyl"). Exemplary C₃₋₆ carbocyclyl groups include, but are not limited to, cyclopropyl (C₃), cyclopropenyl (C₃), cyclobutyl (C₄), cyclobutenyl (C₄), cyclopentyl (Cs), cyclopentenyl (Cs), cyclohexyl (C₆), cyclohexenyl (C₆), cyclohexadienyl (C₆), and the like. Exemplary C₅₋₈ carbocyclyl groups include, but are not limited to, the aforementioned C₃₋₆ carbocyclyl groups as well as cycloheptyl (C₇), cycloheptenyl (C₇), cycloheptadienyl (C₇), cycloheptatrienyl (C₇), cyclooctyl (Cs), cyclooctenyl (Cs), bicyclo[2.2.1]heptanyl (C₇), bicyclo[2.2.2]octanyl (Cs), and the like. As illustrated by the foregoing examples, in some embodiments, the carbocyclyl group is monocyclic ("monocyclic carbocyclyl") or a fused (fused cyclic group), bridged (bridged cyclic group), or spiro-fused (spirocyclic group) ring system such as a bicyclic system ("bicyclic carbocyclyl"), and may be saturated or partially unsaturated. The "carbocyclyl" also encompasses ring systems in which the carbocyclyl ring defined above is fused to one or more aryl or heteroaryl groups, where the point of attachment resides on the carbocyclyl ring; under such circumstances, the ring size of the carbocyclyl ring system corresponds to the number of carbon atoms of the carbocyclic system after fusion. In some embodiments, each example of carbocyclyl groups is independently optionally substituted, i.e., unsubstituted ("unsubstituted carbocyclyl") or substituted with one or more substituents ("substituted carbocyclyl"). In some embodiments, the carbocyclyl group is unsubstituted C₅₋₈ carbocyclyl. In some embodiments, the carbocyclyl group is substituted C₅₋₈ carbocyclyl.

Except as otherwise specified, the term "alkyl" refers to a monovalent saturated aliphatic hydrocarbon group which is a linear or branched group containing 1 to 20 carbon atoms, preferably 1 to 10 carbon atoms (i.e., C₁₋₁₀ alkyl), further preferably 1 to 8 carbon atoms (C₁₋₈ alkyl), and more preferably 1 to 6 carbon atoms (i.e., C₁₋₆ alkyl). For example, "C₁₋₆ alkyl" means an alkyl group having from 1 to 6 (specifically 1, 2, 3, 4, 5 or 6) carbon atoms on the carbon chain. Examples include, but are not limited to, methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl and the like.

Except as otherwise specified, the term "cycloalkyl" refers to a monocyclic saturated aliphatic hydrocarbon group having the specified number of carbon atoms, preferably containing 3 to 12 carbon atoms (i.e., C₃₋₁₂ cycloalkyl), more preferably 3 to 10 carbon atoms (C₃₋₁₀ cycloalkyl), and further preferably 3 to 8 carbon atoms (C₃₋₈ cycloalkyl), 3 to 6 carbon atoms (C₃₋₆ cycloalkyl) or 5 to 6 carbon atoms (C₅₋₆ cycloalkyl). Examples include, but are not limited to, cyclopropyl, cyclobutyl, cyclopentyl, cyclohexyl, methylcyclopropyl, 2-ethylcyclopentyl, dimethylcyclobutyl and the like.

Except as otherwise specified, "cycloalkenyl" is composed of monocyclic hydrocarbon rings, bicyclic hydrocarbon rings and spiro-hydrocarbon rings, whereas the system is unsaturated, containing at least one C=C double bond and free of aromatic moieties. Preferably, cycloalkenyl contains 3 to 12 carbon atoms (i.e., C₃₋₁₂ cycloalkenyl), more preferably 3 to 10 carbon atoms (C₃₋₁₀ cycloalkenyl), and further preferably 3 to 6 carbon atoms (C₃₋₆ cycloalkenyl), 4 to 6 carbon atoms (C₄₋₆ cycloalkenyl), or 5 to 6 carbon atoms (C₅₋₆ cycloalkenyl).

Except as otherwise specified, the term "alkoxy" refers to an -O-alkyl group, wherein alkyl is as defined above, namely containing 1 to 20 carbon atoms, preferably 1 to 10 carbon atoms, more preferably 1 to 8 carbon atoms, and most preferably 1 to 6 (specifically 1, 2, 3, 4, 5 or 6) carbon atoms. Representative examples include, but are not limited to, methoxy, ethoxy, propoxy, isopropoxy, butoxy, 1-methylpropoxy, 2-methylpropoxy, tert-butoxy, pentyloxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy and the like.

Except as otherwise specified, the term "halogen" or "halo" denotes F, Cl, Br or I. The term "haloalkyl" refers to an alkyl group as defined above in which one, two, multiple or all hydrogen atoms are substituted with halogen atoms. Representative examples of haloalkyl include CCl₃, CF₃, CHF₂, CH₂F, CHCl₂, CH₂Cl, CH₂Br, CH₂I, CH₂CF₃, CF₂CF₃ and the like.

Except as otherwise specified, the term "heterocyclyl" or "heterocycle" refers to a saturated or partially unsaturated monocyclic or polycyclic non-aromatic cyclic substituent containing ring carbon atoms and 1 to 4 ring heteroatoms, with 3 to 20 ring atoms total, wherein 1, 2, 3 or more ring atoms are selected from N, O and S, and the remaining ring atoms are C atoms; one or more ring S atom(s) (if present) and/or one or more ring N atom(s) (if present) may be optionally oxidized, and one or more ring carbon atom(s) may be optionally oxidized (i.e., to form an oxo group). Preferably, it contains 3 to 10 ring atoms (3- to 10-membered heterocyclyl), further preferably 3 to 8 ring atoms (3- to 8-membered heterocyclyl), or 3 to 6 ring atoms (3- to 6-membered heterocyclyl), or 4 to 6 ring atoms (4- to 6-membered heterocyclyl), 5 to 8 ring atoms (5- to 8-membered heterocyclyl), or 5 to 6 ring atoms (5- to 6-membered heterocyclyl). The number of heteroatoms is preferably 1 to 4, more preferably 1 to 3 (namely 1, 2 or 3 heteroatoms). Examples of monocyclic heterocyclyl include pyrrolidinyl, imidazolidinyl, tetrahydrofuranyl, dihydropyrrolyl, piperidinyl, piperazinyl, pyranyl and the like. Polycyclic heterocyclyl includes spiro, fused and bridged heterocyclyl groups. "Heterocyclyl" may be monocyclic ("monocyclic heterocyclyl"), or a fused ("fused heterocyclyl" or "hetero-fused cyclic group"), bridged ("bridged heterocyclyl" or "hetero-bridged cyclic group") or spiro-fused ("hetero-spiro cyclic group" or "spiro heterocyclyl") ring system, such as a bicyclic system ("bicyclic heterocyclyl"), and may be saturated or partially unsaturated. A bicyclic heterocyclyl system may contain one or more heteroatoms in one or two rings. "Heterocyclyl" also encompasses ring systems wherein a heterocyclyl ring defined above is fused to one or more carbocyclyl groups, with the point of attachment located on either the carbocyclyl or heterocyclyl ring; alternatively, "heterocyclyl" also includes ring systems wherein the above-defined heterocyclyl ring is fused to one or more aryl or heteroaryl groups, or wherein the cycloalkyl ring defined above is fused to one or more heteroaryl groups, with the point of attachment on the heterocyclyl or cycloalkyl ring. In such cases, the ring size of the heterocyclyl ring system is defined by the number of atoms of the fused ring system. In some embodiments, each example of heterocyclyl is independently optionally substituted, i.e., unsubstituted ("unsubstituted heterocyclyl") or substituted with one or more substituents ("substituted heterocyclyl"). Exemplary 3-membered heterocyclyl groups containing 1 heteroatom include, but are not limited to, aziridinyl, oxiranyl and thiorenyl. Exemplary 4-membered heterocyclyl groups containing 1 heteroatom include, but are not limited to, azetidinyl, oxetanyl and thietanyl. Exemplary 5-membered heterocyclyl groups containing 1 heteroatom include, but are not limited to, tetrahydrofuranyl, dihydrofuranyl, tetrahydrothiophenyl, dihydrothiophenyl, pyrrolidinyl, dihydropyrrolyl and pyrrolidine-2,5-dionyl. Exemplary 5-membered heterocyclyl groups containing 2 heteroatoms include, but are not limited to, dioxolanyl, oxathiolanyl, dithiolanyl and oxazolidin-2-onyl. Exemplary 5-membered heterocyclyl groups containing 3 heteroatoms include, but are not limited to, triazolinyl, oxadiazolinyl and thiadiazolinyl. Exemplary 6-membered heterocyclyl groups containing 1 heteroatom include, but are not limited to, piperidinyl, tetrahydropyranyl, dihydropyridinyl and thianyl. Exemplary 6-membered heterocyclyl groups containing 2 heteroatoms include, but are not limited to, piperazinyl, morpholinyl, dithianyl and dioxanyl. Exemplary 6-membered heterocyclyl groups containing 3 heteroatoms include, but are not limited to, triazinanyl, oxadiazinanyl, thiadiazinanyl, oxathiazinanyl and dioxazinanyl. Exemplary 7-membered heterocyclyl groups containing 1 heteroatom include, but are not limited to, azepanyl, oxepanyl and thiepanyl. Exemplary 8-membered heterocyclyl groups containing 1 heteroatom include, but are not limited to, azocanyl, oxocanyl and thiocanyl. An exemplary 5-membered heterocyclyl group fused to a C₆ aryl ring (also referred to herein as a 5,6-bicyclic heterocycle) includes, but is not limited to, indolinyl, isoindolinyl, dihydrobenzofuranyl, dihydrobenzothiophenyl, benzoxazolinonyl and the like. An exemplary 6-membered heterocyclyl group fused to an aryl ring (also referred to herein as a 6,6-bicyclic heterocycle) includes, but is not limited to, tetrahydroquinolinyl, tetrahydroisoquinolinyl and the like.

Except as otherwise specified, the term "heterocycloalkyl" refers to a monocyclic saturated "heterocyclyl" or "heterocycle" as defined above, with ring atoms as defined above, i.e., containing 3 to 20 ring atoms ("3- to 20-membered heterocycloalkyl") and 1 to 4 (1, 2, 3 or 4) heteroatoms, preferably 1 to 3 (1, 2 or 3) heteroatoms, each heteroatom independently selected from N, O, and S. One or more ring S atom(s) (if present) and/or one or more ring N atom(s) (if present) may be optionally oxidized, and one or more ring carbon atom(s) may be optionally oxidized (i.e., to form an oxo group). Heterocycloalkyl preferably contains 3 to 12 ring atoms ("3- to 12-membered heterocycloalkyl"), further preferably 3 to 10 ring atoms ("3- to 10-membered heterocycloalkyl"), still further preferably 3 to 8 ring atoms ("3- to 8-membered heterocycloalkyl"), and more further preferably 3 to 4 ring atoms ("3- to 4-membered heterocycloalkyl"). In some embodiments, each example of heterocycloalkyl is independently optionally substituted, i.e., unsubstituted ("unsubstituted heterocycloalkyl") or substituted with one or more substituents ("substituted heterocycloalkyl"). Exemplary heterocyclylalkyl groups have been partially listed in the foregoing section relating to "heterocyclyl" or "heterocycle", which include, but are not limited to, aziridinyl, oxiranyl, thiiranyl, azetidinyl, oxetanyl, thietanyl, tetrahydrofuranyl, oxanyl, piperidinyl, piperazinyl, morpholinyl, thiomorpholinyl, oxathianyl, oxazolidinyl, dioxanyl, dithianyl, thiazolidinyl, pyrrolidinyl, pyrazolidinyl, imidazolidinyl and the like.

Except as otherwise specified, the term "heterocycloalkenyl" refers to an unsaturated alicyclic (non-aromatic) cyclic group including monocyclic, bridged, spiro and/or fused ring systems (which may consist of two or three rings; for example, fused ring systems composed of two or three fused rings). The cyclic group contains one or more (e.g., one, two, three or four) ring heteroatoms each independently selected from O, S and N, with the remaining ring atoms being carbon atoms. One or more ring S atom(s) (if present) and/or one or more ring N atom(s) (if present) may be optionally oxidized, and one or more ring carbon atom(s) may be optionally oxidized (i.e., to form an oxo group). In addition, the cyclic group bears at least one double bond between adjacent ring atoms and is free of any triple bonds between adjacent ring atoms. By way of example, each heteroatom-containing ring comprised in the unsaturated alicyclic cyclic group may contain one or two O atoms and/or one or two S atoms (optionally oxidized) and/or one, two, three or four N atoms (optionally oxidized), provided that the total number of heteroatoms in the respective heteroatom-containing ring ranges from 1 to 4 and the respective heteroatom-containing ring contains at least one ring carbon atom (optionally oxidized). Exemplary "heterocycloalkenyl" groups include imidazolinyl (e.g., 2-imidazolinyl (i.e., 4,5-dihydro-1H-imidazolyl), 3-imidazolinyl or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably denotes a 3- to 12-membered unsaturated alicyclic group which is a monocyclic or fused ring system (e.g., a fused ring system formed of two fused rings), containing one or more (e.g., one, two, three or four) ring heteroatoms independently selected from O, S and N; wherein one or more ring S atom(s) (if present) and/or one or more ring N atom(s) (if present) are optionally oxidized, one or more ring carbon atom(s) are optionally oxidized, the cyclic group contains at least one double bond between adjacent ring atoms and contains no triple bonds between adjacent ring atoms. More preferably, "heterocycloalkenyl" refers to a 5- to 7-membered monocyclic unsaturated non-aromatic cyclic group bearing one or more (e.g., one, two or three) ring heteroatoms independently selected from O, S and N, wherein one or more ring S atom(s) (if present) and/or one or more ring N atom(s) (if present) are optionally oxidized, one or more ring carbon atom(s) are optionally oxidized, and the cyclic group contains at least one double bond but no triple bonds between adjacent ring atoms.

Except as otherwise specified, the term "aryl" or "aromatic carbocyclyl" denotes monocyclic, bicyclic or tricyclic aromatic carbocyclic ring systems containing 6 to 16 carbon atoms, alternatively 6 to 14 carbon atoms, alternatively 6 to 12 carbon atoms, alternatively 6 to 10 carbon atoms, preferably 6 to 10 carbon atoms. The term "aryl" may be used interchangeably with "aromatic cyclic group". Exemplary aryl groups include, but are not limited to, phenyl, naphthyl, anthracenyl, phenanthrenyl, or pyrenyl, etc.

Except as otherwise specified, the term "heteroaryl" or "aromatic heterocyclyl" refers to monocyclic or polycyclic aromatic ring systems having a 5- to 14-membered skeleton, preferably 5- to 10-membered, more preferably 5- to 8-membered, most preferably 5- to 6-membered, wherein 1, 2, 3 or more ring atoms are heteroatoms and the remaining ring atoms are carbon, each heteroatom is independently selected from O, N, and S, and the number of heteroatoms is preferably 1, 2 or 3. Examples of heteroaryl include, but are not limited to, furyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidinyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuranyl, benzothienyl, benzopyridyl, benzopyrimidinyl, benzopyrazinyl, benzimidazolyl, benzophthalazinyl, pyrrolo[2,3-b]pyridinyl, imidazo[1,2-a]pyridinyl, pyrazolo[1,5-a]pyridinyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl, etc.

Except as otherwise specified, the term "treatment" encompasses any treatment of a disease, disorder or condition in a patient, including: (a) inhibiting symptoms of the disease, disorder or condition, i.e., arresting its progression; (b) alleviating symptoms of the disease, disorder or condition, i.e., causing regression of the disease or symptoms; or (c) ameliorating or eliminating the disease, disorder or condition or one or more symptoms associated therewith.

As used herein, a therapeutically effective amount refers to a pharmaceutically efficacious dosage for administration, namely an amount of an active compound sufficient to achieve a marked improvement in disease conditions without inducing severe adverse side effects.

### Beneficial effects of the present Invention:

The present invention provides an MAO-B inhibitor with a novel structure. In-vitro experimental results show that the compound in the present invention has relatively high MAO-B selective inhibition activity. In-vivo experimental results show that the compound in the present invention can remarkably reduce the weight of a mouse under the condition that the feeding amount is not affected.

### BRIEF DESCRIPTION OF THE DRAWINGS

FIG. 1 shows changes in weight of DIO mice in a weight-loss experiment.
FIG. 2 shows changes in weight gain rate of DIO mice in the weight-loss experiment.
FIG. 3 shows changes in food intake of DIO mice in the weight-loss experiment.

### DETAILED DESCRIPTION

The present invention is further illustrated with reference to specific examples below. It should be understood that these examples are merely illustrative of the present invention and shall not be construed as limiting the scope thereof. Unless otherwise specified, experimental procedures in the following examples are carried out under conventional conditions or conditions recommended by the respective equipment manufacturers. All technical and scientific terms used herein have the same meaning as commonly understood by those skilled in the art unless otherwise defined. In addition, any methods and materials similar or equivalent to those described herein can also be employed in the methods of the present invention. The preferred methods and materials described below are provided for exemplary purposes only.

The structures of the compounds according to the present invention are characterized by nuclear magnetic resonance (NMR), liquid chromatography-mass spectrometry (LC-MS) and/or high-performance liquid chromatography (HPLC). NMR analysis is performed on Bruker AVANCE III 600 MHz; LC-MS analysis is performed on LCMS WATERS ACQUITY UPLC H-Class PLUS or/and SQD2; HPLC testing is conducted using WATERS e2695_2998 or/and Agilent 1100.

Starting materials used in the examples of the present invention are well known and commercially available, or can be synthesized via conventional methods known in the art.

Glossary of terms or abbreviations:
OMs: methanesulfonyl
OTf: trifluoromethanesulfonyl
OTs: p-toluenesulfonyl
B(OH)₂: boronic acid group
Bpin: pinacolborate
Bneop: neopentylborate

### Example 1: Preparation of compound 1

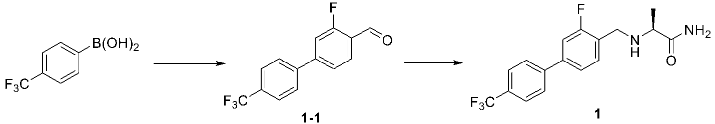

### (1) Preparation of intermediate 1-1

2-Fluoro-4-bromobenzaldehyde (203 mg, 1.00 mmol), 4-(trifluoromethyl)phenylboronic acid (247 mg, 1.30 mmol), sodium carbonate (424 mg, 4.00 mmol) and tetrakis(triphenylphosphine)palladium (58 mg, 0.05 mmol) were added into toluene/methanol/water (4 mL/2 mL/1 mL). The reaction system was purged with nitrogen 3 times, heated to 100 °C under nitrogen atmosphere and stirred to react for 18 h. The mixture was filtered through diatomaceous earth, extracted with ethyl acetate (20 mL ×3), and the combined organic phases were washed with saturated sodium chloride solution (20 mL × 2), dried over anhydrous sodium sulfate and concentrated under reduced pressure. The residue was purified by column chromatography (petroleum ether/ethyl acetate = 50:1) to afford intermediate 1-1 (231 mg, yield 86%).

### (2) Preparation of compound 1

Intermediate 1-1 (231 mg, 0.86 mmol) was dissolved in anhydrous methanol (10 mL), followed by addition of L-alaninamide hydrochloride (128 mg, 1.03 mmol) and triethylamine (130 mg, 1.29 mmol). The resultant mixture was allowed to react at room temperature for 2 h, cooled to 0 °C, and sodium cyanoborohydride (216 mg, 3.44 mmol) was added. The reaction mixture was then gradually warmed to room temperature and allowed to react overnight. After concentration under reduced pressure, the mixture was purified by preparative thin-layer chromatography (dichloromethane/methanol = 20:1) to give compound 1 (228 mg, yield 78%). **ESI-MS** (m/z): 341.15 [M+H]⁺. ¹H NMR (600 MHz, DMSO-*d*₆) δ 7.92 (d, *J =* 8.4 Hz, 2H), 7.81 (d, *J =* 8.4 Hz, 2H), 7.62-7.56 (m, 3H), 7.33 (s, 1H), 7.02 (s, 1H), 3.75 (d, *J =* 14.4 Hz, 1H), 3.65 (d, *J =* 14.4 Hz, 1H), 3.06 (q, *J =* 6.6 Hz, 1H), 1.16 (d, *J =* 6.6 Hz, 3H).

### Example 2: Preparation of compound 2

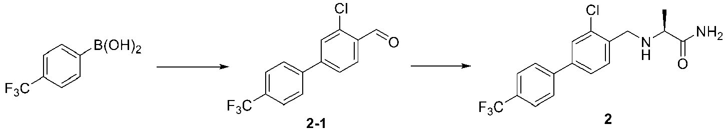

Compound 2 (222 mg, 62% yield over 2 steps) was prepared following the preparation method for compound 1 by replacing 2-fluoro-4-bromobenzaldehyde with 2-chloro-4-bromobenzaldehyde. ESI-MS (m/z): 357.16 [M+H]⁺. ¹H NMR (600 MHz, DMSO-*d*₆) δ 7.92 (d, *J* = 7.8 Hz, 2H), 7.81-7.79 (m, 3H), 7.71 (d, *J =* 8.4 Hz, 1H), 7.68 (d, *J =* 7.8 Hz, 1H), 7.35 (s, 1H), 7.02 (s, 1H), 3.80 (d, *J* = 15.0 Hz, 1H), 3.70 (d, *J =* 15.0 Hz, 1H), 3.09 (q, *J =* 6.6 Hz, 1H), 2.54 (s, 1H), 1.19 (d, *J =* 6.6 Hz, 3H).

### Example 3: Preparation of compound 3

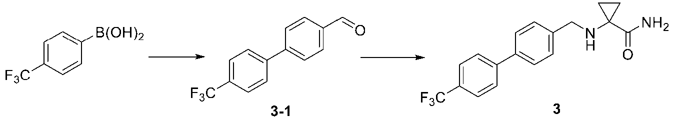

Compound 3 (191 mg, 57% yield over 2 steps) was prepared according to the preparation method for compound 1 by replacing 2-fluoro-4-bromobenzaldehyde with 4-bromobenzaldehyde and replacing L-alaninamide hydrochloride with 1-aminocyclopropanecarboxamide. **ESI-MS** (m/z): 335.16 [M+H]⁺. ¹H NMR (600 MHz, DMSO-*d*₆) δ 7.88 (d, *J =* 8.4 Hz, 2H), 7.80 (d, *J =* 8.4 Hz, 2H), 7.68 (d, *J =* 7.8 Hz, 2H), 7.48 (d, *J =* 7.8 Hz, 2H), 7.47 (s, 1H), 7.16 (s, 1H), 3.74 (s, 2H), 2.86 (s, 1H), 1.05-1.04 (m, 2H), 0.90-0.87 (m, 2H).

### Example 4: Preparation of compound 4

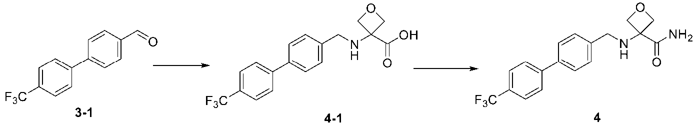

### (1) Preparation of intermediate 4-1

Intermediate 4-1 (222 mg, yield 61%) was prepared following the preparation method for compound 3 by replacing 1-aminocyclopropanecarboxamide with 3-aminooxetane-3-carboxylic acid.

### (2) Preparation of compound 4

Intermediate 4-1 (80 mg, 0.23 mmol) was dissolved in dichloromethane (10 mL), then 2-(7-azabenzotriazole)-N,N,N',N'-tetramethyluronium hexafluorophosphate (175 mg, 0.46 mmol), ammonium chloride (37 mg, 0.69 mmol) and triethylamine (70 mg, 0.69 mmol) were added sequentially. The mixture was allowed to react at room temperature for 4 h. The mixture was concentrated under reduced pressure to afford a crude product, which was purified via column chromatography (dichloromethane: methanol = 50:1 to 20:1) to give compound 4 (40 mg, yield 50%). **ESI-MS** (m/z): 351.10 [M+H]⁺. ¹H NMR (600 MHz, DMSO-*d*₆) δ 7.90 (d, *J =* 8.4 Hz, 2H), 7.81 (d, *J* = 8.4 Hz, 2H), 7.71 (d, *J =* 8.4 Hz, 2H), 7.53 (d, *J =* 8.4 Hz, 2H), 7.37 (s, 1H), 7.28 (s, 1H), 4.72 (d, *J =* 6.0 Hz, 2H), 4.43 (d, *J =* 6.0 Hz, 2H), 3.59 (d, *J =* 7.2 Hz, 2H), 3.37 (t, *J =* 7.2 Hz, 1H).

### Example 5: Preparation of compound 5

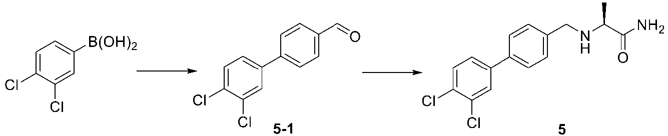

Compound 5 (239 mg, 74% yield over 2 steps) was prepared according to the preparation method for compound **1** by replacing 4-(trifluoromethyl)phenylboronic acid with 3,4-dichlorophenylboronic acid and 2-fluoro-4-bromobenzaldehyde with 4-bromobenzaldehyde. **ESI-MS** (m/z): 323.10 [M+H]⁺. ¹H NMR (600 MHz, DMSO-*d*₆) δ 7.92 (d, *J =* 1.8 Hz, 1H), 7.71-7.66 (m, 4H), 7.44 (d, *J* = 8.4 Hz, 2H), 7.34 (s, 1H), 6.99 (s, 1H), 3.73 (d, *J* = 13.8 Hz, 1H), 3.59 (d, *J* = 13.8 Hz, 1H), 3.03 (q, *J* = 7.2 Hz, 1H), 1.15 (d, *J* = 6.6 Hz, 3H).

### Example 6: Preparation of compound 6

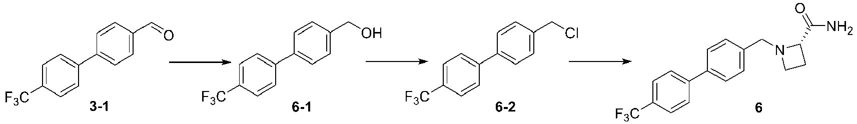

### (1) Preparation of intermediate 6-1

Intermediate **3-1** (250 mg, 1.00 mmol) was dissolved in anhydrous methanol (10 mL), followed by the addition of sodium borohydride (45 mg, 1.20 mmol), and the mixture was stirred to react at room temperature for 1 h. Water (20 mL) was added to the reaction mixture, and the mixture was concentrated and extracted with ethyl acetate (20 mL × 3). The organic phases were combined, washed with saturated sodium chloride solution (20 mL × 2), dried over anhydrous sodium sulfate, concentrated under reduced pressure, and purified by column chromatography (petroleum ether: ethyl acetate = 50:1-10:1) to give intermediate 6-1 (250 mg, yield 99%).

### (2) Preparation of intermediate 6-2

Intermediate 6-1 (250 mg, 0.99 mmol) was dissolved in anhydrous N,N-dimethylformamide (10 mL), and thionyl chloride (118 mg, 1.98 mmol) was added at 0 °C. The system was allowed to react at room temperature for 2 h. Water (20 mL) was added to the reaction mixture, and the mixture was extracted with ethyl acetate (20 mL ×3). The organic phases were combined, washed with saturated sodium chloride solution (30 mL × 2), dried over anhydrous sodium sulfate, concentrated under reduced pressure, and purified by column chromatography (petroleum ether: ethyl acetate = 50:1) to give intermediate 6-2 (267 mg, yield 99%).

### (3) Preparation of compound 6

Intermediate 6-2 (267 mg, 0.98 mmol), (S)-azetidine-2-carboxamide (118 mg, 1.18 mmol) and triethylamine (148 mg, 1.47 mmol) were dissolved in N,N-dimethylformamide (10 mL), and the reaction mixture was allowed to react overnight at room temperature. Upon addition of water (20 mL), the mixture was extracted with ethyl acetate (20 mL ×3). The combined organic phases were washed with saturated sodium chloride solution (20 mL × 2), dried over anhydrous sodium sulfate and concentrated under reduced pressure. Purification by column chromatography (dichloromethane: methanol = 50:1 to 20:1) provided compound 6 (134 mg, yield 41%). ESI-MS (m/z): 335.20 [M+H]⁺. ¹HNMR (600 MHz, CDCl₃) δ 7.70-7.67 (m, 4H), 7.56 (d, *J* = 7.2 Hz, 2H), 7.37 (d, *J* = 7.2 Hz, 2H), 7.05 (s,1H), 5.57 (s,1H), 3.79 (d, *J* = 12.6 Hz, 1H), 3.72 (dd, *J* = 8.4, 8.4Hz, 1H), 3.62 (d, *J =* 12.6 Hz, 1H), 3.41-3.38 (m, 1H), 3.06-3.02 (m, 1H), 2.47-2.42 (m, 1H), 2.24-2.17 (m, 1H).

### Example 7: Preparation of compound 7

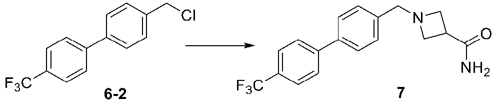

Compound 7 (80 mg, yield 64%) was prepared following the preparation method for compound 6 by replacing (S)-azetidine-2-carboxamide with azetidine-3-carboxamide hydrochloride. ESI-MS (m/z): 335.06 [M+H]⁺. ¹H NMR (600 MHz, DMSO-*d*₆) δ 7.89 (d, *J =* 7.8 Hz, 2H), 7.80 (d, *J =* 8.4 Hz, 2H), 7.69 (d, *J =* 8.4 Hz, 2H), 7.41 (d, *J =* 7.8 Hz, 2H), 7.31 (s, 1H), 6.90 (s, 1H), 3.62 (s, 2H), 3.41-3.39 (m, 2H), 3.21-3.19 (m, 2H), 3.17-3.11 (m, 1H).

### Example 8: Preparation of compound 8

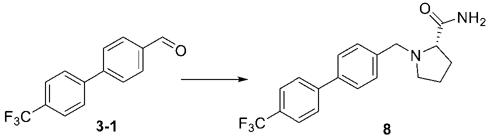

Compound 8 (153 mg, yield 44%) was prepared following the preparation method for compound 3 by replacing 1-aminocyclopropanecarboxamide with L-prolinamide. **ESI-MS** (m/z): 349.12 [M+H]⁺. ¹HNMR (600 MHz, CDCl₃) δ 7.69-7.68 (m, 4H), 7.56 (d, *J* = 7.8 Hz, 2H), 7.39 (d, *J* = 7.8 Hz, 2H), 7.24 (s,1H), 5.45 (s,1H), 4.00 (d, *J =* 13.2 Hz, 1H), 3.55 (d, *J =* 13.2 Hz, 1H), 3.24-3.21 (m, 1H), 3.10-3.08 (m, 1H), 2.41-2.37 (m,1H), 2.31-2.24 (m,1H), 1.98-1.93 (m,1H), 1.86-1.76 (m, 2H).

### Example 9: Preparation of compound 9

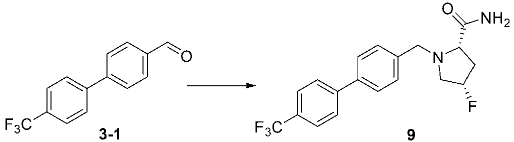

Compound 9 (132 mg, yield 36%) was prepared following the preparation method for compound 3 by replacing L-alaninamide hydrochloride with (2S,4S)-4-fluoropyrrolidine-2-carboxamide. ESI-MS (m/z): 367.16 [M+H]⁺. ¹HNMR (600 MHz, CDCl₃) δ 7.71-7.68 (m, 4H), 7.58 (d, *J* = 7.8 Hz, 2H), 7.40(d, *J* = 7.8 Hz, 2H), 7.25 (s,1H), 5.37 (s,1H), 5.37 (ddd, *J* = 53.4, 3.6, 3.6 Hz, 1H), 4.07 (d, *J* = 13.2 Hz, 1H), 3.57 (d, *J* = 13.2 Hz, 1H), 3.38-3.30 (m, 2H), 2.67-2.50 (m,2H), 2.31-2.24 (m,1H).

### Example 10: Preparation of compound 10

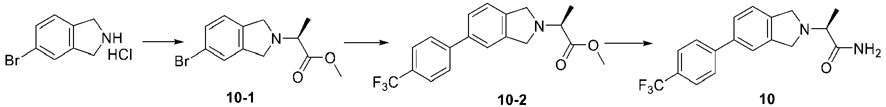

### (1) Preparation of intermediate 10-1

5-Bromoisoindoline hydrochloride (1.00 g, 4.26 mmol), methyl (R)-2-bromopropanoate (1.07 g, 6.40 mmol) and N,N-diisopropylethylamine (1.65 g, 12.78 mmol) were dissolved in dichloromethane (30 mL), and the system was allowed to react overnight at room temperature. Water (50 mL) was added, and the mixture was extracted with ethyl acetate (50 mL × 3). The combined organic phases were washed with saturated sodium chloride (50 mL × 2), dried over anhydrous sodium sulfate and concentrated under reduced pressure. Purification via column chromatography (petroleum ether: ethyl acetate = 20:1 to 5:1) afforded intermediate 10-1 (900 mg, yield 74%).

### (2) Preparation of intermediate 10-2

Intermediate 10-1 (284 mg, 1.00 mmol), 4-(trifluoromethyl)phenylboronic acid (247 mg, 1.30 mmol), sodium carbonate (424 mg, 4.00 mmol) and tetrakis(triphenylphosphine)palladium (58 mg, 0.05 mmol) were added into toluene/methanol/water (4 mL/2 mL/1 mL). The reaction system was purged with nitrogen 3 times, heated to 100 °C under nitrogen atmosphere and stirred to react for 18 h. The mixture was filtered through diatomaceous earth, extracted with ethyl acetate (20 mL × 3), and the combined organic phases were washed with saturated sodium chloride (20 mL × 2), dried over anhydrous sodium sulfate and concentrated under reduced pressure. The residue was purified by column chromatography (petroleum ether/ethyl acetate = 20:1 to 5:1) to afford intermediate 10-2 (241 mg, yield 69%).

### (3) Preparation of compound 10

Intermediate 10-2 (241 mg, 0.69 mmol) and sodium hydroxide (42 mg, 1.04 mmol) were dissolved in tetrahydrofuran/methanol/water (10 mL/2 mL/2 mL), and the system was allowed to react at 50 C for 6 h. Water (10 mL) was added, and the mixture was adjusted to pH 5-6 prior to extraction with dichloromethane (20 mL × 3). The combined organic phases were washed with saturated sodium chloride (20 mL × 2), dried over anhydrous sodium sulfate and concentrated under reduced pressure. The resulting residue was dissolved in dichloromethane (10 mL), then 2-(7-azabenzotriazole)-N,N,N',N'-tetramethyluronium hexafluorophosphate (394 mg, 1.04 mmol), ammonium chloride (55 mg, 1.04 mmol) and triethylamine (139 mg, 1.38 mmol) were added. The reaction mixture was allowed to react at room temperature for 4 h and concentrated under reduced pressure. Purification by column chromatography (dichloromethane: methanol = 50:1 to 20:1) yielded compound 10 (100 mg, yield 43%). ESI-MS (m/z): 335.16 [M+H]⁺. ¹H NMR (600 MHz, DMSO-*d*₆) δ 7.86 (d, *J* = 8.4 Hz, 2H), 7.80 (d, *J* = 8.4 Hz, 2H), 7.62 (s, 1H), 7.57-7.56 (m, 1H), 7.38 (d, *J* = 7.8 Hz, 1H), 7.32 (s, 1H), 7.02 (s, 1H), 4.02-3.99 (m, 4H), 3.20 (q, *J* = 6.6 Hz, 1H), 1.29 (d, *J* = 6.6 Hz, 3H).

### Example 11: Preparation of compound 11

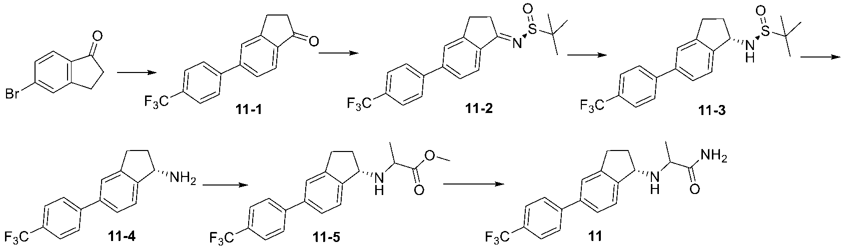

### (1) Preparation of intermediate 11-1

5-Bromo-1-indanone (1.00 g, 4.74 mmol), 4-(trifluoromethyl)phenylboronic acid (1.15 g, 6.07 mmol), sodium carbonate (2.44 g, 23.04 mmol), and tetrakis(triphenylphosphine)palladium (0.27 g, 0.24 mmol) were dissolved in toluene/water (21 mL/3 mL), purged with nitrogen 3 times, and heated to 110 °C under nitrogen atmosphere with stirring to react for 4 h. The mixture was filtered through diatomaceous earth and extracted with ethyl acetate (20 mL ×3). The combined organic phases were washed with saturated aqueous sodium chloride solution (20 mL × 2), dried over anhydrous sodium sulfate and concentrated under reduced pressure. Purification by column chromatography (petroleum ether: ethyl acetate = 20:1 to 5:1) afforded intermediate 11-1 (1.25 g, yield 95%).

### (2) Preparation of intermediate 11-2

Intermediate 11-1 (450 mg, 1.63 mmol), (S)-tert-butanesulfinamide (237 mg, 1.96 mmol) and tetraethyl titanate (1.10 g, 4.89 mmol) were dissolved in tetrahydrofuran (20 mL), and the resulting mixture was heated to 80 C to react with stirring for 24 h. Water (20 mL) was added to the reaction mixture, followed by filtration through diatomaceous earth. The filter cake was rinsed with ethyl acetate, the solution was collected, and extracted with ethyl acetate (20 mL × 3). The organic phases were combined, washed with saturated aqueous sodium chloride solution (20 mL ×2), dried over anhydrous sodium sulfate, concentrated under reduced pressure, and purified by column chromatography (petroleum ether/ethyl acetate = 10:1 to 2:1) to give intermediate 11-2 (220 mg, yield 36%).

### (3) Preparation of intermediate 11-3

Intermediate 11-2 (220 mg, 0.58 mmol) was dissolved in tetrahydrofuran (20 mL), and sodium borohydride (44 mg, 1.16 mmol) was slowly added. The mixture was stirred to react at room temperature for 3 h. After concentration under reduced pressure, the residue was purified via column chromatography (petroleum ether/ethyl acetate = 5:1 to 1:1) to give intermediate 11-3 (170 mg, yield 77%).

### (4) Preparation of intermediate 11-4

Intermediate 11-3 (170 mg, 0.45 mmol) was dissolved in dioxane hydrochloride (10 mL, 2 M), and the mixture was stirred to react at room temperature for 2 h. The mixture was concentrated under reduced pressure to give intermediate 11-4, which was used directly in the next reaction.

### (5) Preparation of intermediate 11-5

Intermediate 11-4, methyl 2-chloropropionate (66 mg, 0.54 mmol), potassium carbonate (186 mg, 1.35 mmol), and potassium iodide (75 mg, 0.45 mmol) were dissolved in N,N-dimethylformamide (15 mL), and the mixture was stirred at 90 °C to react for 24 h. Water (20 mL) was added, and the mixture was extracted with ethyl acetate (20 mL ×3). The combined organic layers were washed with saturated aqueous sodium chloride solution (20 mL × 2), dried over anhydrous sodium sulfate and concentrated under reduced pressure. Purification via column chromatography (petroleum ether: ethyl acetate = 10:1 to 3:1) provided Intermediate 11-5 (60 mg, yield 37%).

### (6) Preparation of compound 11

Intermediate 11-5 (60 mg, 0.17 mmol) and sodium hydroxide (34 mg, 0.85 mmol) were dissolved in tetrahydrofuran/methanol/water (2 mL/2 mL/2 mL) and allowed to react at room temperature for 2 h. Water (5 mL) was added, and the mixture was adjusted to pH 5-6 prior to extraction with dichloromethane (15 mL × 3). The combined organic phases were washed with saturated sodium chloride solution (15 mL × 2), dried over anhydrous sodium sulfate and concentrated under reduced pressure. The resulting residue was dissolved in dichloromethane (10 mL), then 2-(7-azabenzotriazole)-N,N,N',N'-tetramethyluronium hexafluorophosphate (129 mg, 0.34 mmol), ammonium chloride (27 mg, 0.51 mmol) and triethylamine (52 mg, 0.51 mmol) were added. The reaction mixture was allowed to react at room temperature for 4 h and concentrated under reduced pressure. Purification by column chromatography (dichloromethane: methanol = 50:1 to 20:1) yielded compound 11 (20 mg, yield 35%). **ESI-MS** (m/z): 349.12 [M+H]⁺. ¹H NMR (600 MHz, DMSO-*d*₆) δ 7.87 (d, *J* = 8.4 Hz, 2H), 7.80 (d, *J* = 8.4 Hz, 2H), 7.58 (d, *J* = 3.0 Hz, 1H), 7.55-7.52 (m, 1H), 7.46-7.41 (m, 2H), 7.04 (s, 1H), 4.11-4.10 (m, 1H), 3.26-3.19 (m, 1H), 3.03-2.99 (m, 1H), 2.82-2.78 (m, 1H), 2.37-2.34 (m, 1H), 2.32-2.25 (m, 1H), 1.81-1.74 (m, 1H), 1.19-1.17 (m, 3H).

### Example 12: Preparation of compound 12

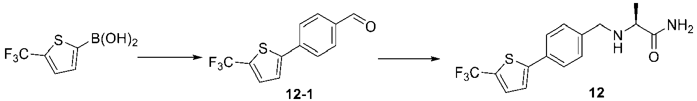

Compound 12 (161 mg, 49% yield over 2 steps) was prepared following the preparation method for compound **1** by replacing 4-(trifluoromethyl)phenylboronic acid with 5-(trifluoromethyl)thiophene-2-boronic acid and 2-fluoro-4-bromobenzaldehyde with 4-bromobenzaldehyde. **ESI-MS** (m/z): 329.12 [M+H]⁺. ¹H NMR (600 MHz, DMSO-*d*₆) δ 7.72 (d, *J* = 3.0 Hz, 1H), 7.69 (d, *J =* 7.8 Hz, 2H), 7.59 (d, *J =* 3.0 Hz, 1H), 7.43 (d, *J =* 8.4 Hz, 2H), 7.34 (s, 1H), 6.99 (s, 1H), 3.73 (d, *J* = 14.4 Hz, 1H), 3.59 (d, *J =* 14.4 Hz, 1H), 3.03 (q, *J* = 6.6 Hz, 1H), 1.15 (d, *J =* 6.6 Hz, 3H).

### Example 13: Preparation of compound 13

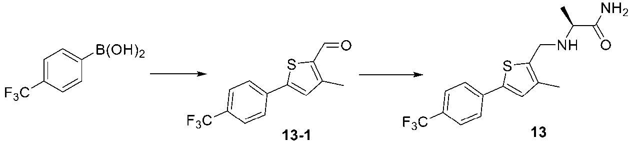

Compound 13 (188 mg, 55% yield over 2 steps) was prepared according to the preparation method for compound **1,** with 2-fluoro-4-bromobenzaldehyde replaced by 3-methyl-5-bromothiophene-2-carboxaldehyde. **ESI-MS** (m/z): 343.12 [M+H]⁺. ¹H NMR (600 MHz, DMSO-*d₆*) δ 7.79 (d, *J =* 8.4 Hz, 2H), 7.72 (d, *J =* 8.4 Hz, 2H), 7.40 (s, 1H), 7.33 (s, 1H), 7.02 (s, 1H), 3.79 (d, *J =* 14.4 Hz, 1H), 3.68 (d, *J =* 14.4 Hz, 1H), 3.12 (q, *J =* 6.6 Hz, 1H), 2.60 (s, 1H), 2.14 (s, 3H), 1.15 (d, *J* = 7.2 Hz, 3H).

### Example 14: Preparation of compound 14

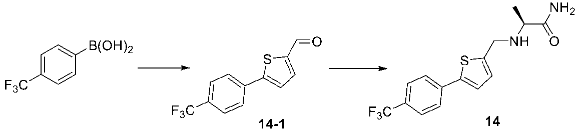

Compound 14(204 mg, 62% yield over 2 steps) was prepared according to the preparation method for compound **1,** with 2-fluoro-4-bromobenzaldehyde replaced by 5-bromothiophene-2-carboxaldehyde. **ESI-MS** (m/z): 329.11 [M+H]⁺. ¹H NMR (600 MHz, DMSO-*d*₆) δ 7.83 (d, *J =* 7.8 Hz, 2H), 7.73 (d, *J =* 7.8 Hz, 2H), 7.52 (d, *J =* 3.6 Hz, 1H), 7.33 (s, 1H), 7.01-6.99 (m, 2H), 3.90 (d, *J* = 15.0 Hz, 1H), 3.76 (d, *J =* 15.0 Hz, 1H), 3.12 (q, *J =* 7.2 Hz, 1H), 2.69 (s, 1H), 1.15 (d, *J =* 6.6 Hz, 3H).

### Example 15: Preparation of compound 15

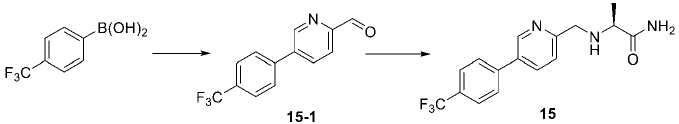

Compound 15 (222 mg, 47% yield over 2 steps) was prepared following the preparation method for compound 1 by substituting 2-fluoro-4-bromobenzaldehyde with 5-bromo-2-pyridinecarboxaldehyde. **ESI-MS** (m/z): 324.13 [M+H]⁺. ¹H NMR (600 MHz, DMSO-*d*₆) δ 8.88 (d, *J =* 1.8 Hz, 1H), 8.15-8.13 (m, 1H), 7.96 (d, *J =* 7.8 Hz, 2H), 7.85 (d, *J =* 8.4 Hz, 2H), 7.60 (d, *J =* 7.8 Hz, 1H), 7.38 (s, 1H), 7.02 (s, 1H), 3.86 (d, *J* = 15.0 Hz, 1H), 3.75 (d, *J =* 15.0 Hz, 1H), 3.10 (q, *J =* 6.6 Hz, 1H), 2.73 (s, 1H), 1.19 (d, *J =* 6.6 Hz, 3H).

### Example 16: Preparation of compound 16

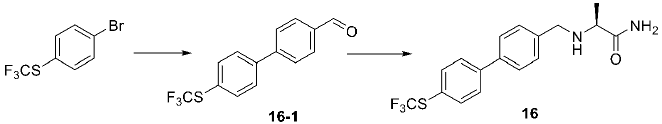

### (1) Preparation of intermediate 16-1

4-(Thiotrifluoromethyl)bromobenzene (300 mg, 1.17 mmol), 4-formylphenylboronic acid (264 mg, 1.76 mmol), sodium carbonate (248 mg, 2.34 mmol) and tetrakis(triphenylphosphine)palladium (139 mg, 0.12 mmol) were added into toluene/methanol/water (18 mL/2 mL/2 mL). The reaction system was purged with nitrogen 3 times, then heated to 110 °C and stirred to react under nitrogen atmosphere for 4 h. The mixture was filtered through diatomaceous earth, extracted with ethyl acetate (20 mL × 3), and the combined organic phases were washed with saturated sodium chloride solution (20 mL × 2), dried over anhydrous sodium sulfate and concentrated under reduced pressure. The residue was purified by column chromatography (petroleum ether: ethyl acetate = 30:1 to 10:1) to afford intermediate 16-1 (300 mg, yield 91%).

### (2) Preparation of compound 16

Compound 16 (20 mg, 32% yield) was prepared following the preparation method for compound 1 by replacing intermediate 1-1 with Intermediate 16-1. **ESI-MS** (m/z): 355.07 [M+H]⁺. ¹H NMR (600 MHz, DMSO-*d*₆) δ 7.84 (d, *J =* 8.4 Hz, 2H), 7.80 (d, *J =* 8.4 Hz, 2H), 7.69 (d, *J =* 8.4 Hz, 2H), 7.47 (d, *J =* 8.4 Hz, 2H), 7.35 (s, 1H), 7.01 (s, 1H), 3.75 (d, *J =* 13.8 Hz, 1H), 3.61 (d, *J =* 13.8 Hz, 1H), 3.05 (q, *J =* 6.6 Hz, 1H), 1.16 (d, *J =* 6.6 Hz, 3H).

### Example 17: Preparation of compound 17

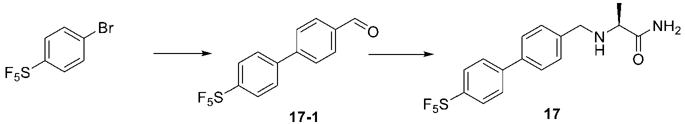

Following the preparation method for compound 16, 4-(thiotrifluoromethyl)bromobenzene was replaced with 4-(pentafluorosulfanyl)bromobenzene to afford compound 17 (20 mg, 36% yield over 2 steps), **ESI-MS** (m/z): 381.06 [M+H]⁺. ¹H NMR (600 MHz, DMSO-*d*₆) δ 7.97 (d, *J* = 9.0 Hz, 2H), 7.89 (d, *J* = 9.0 Hz, 2H), 7.70 (d, *J =* 7.8 Hz, 2H), 7.48 (d, *J =* 7.8 Hz, 2H), 7.35 (s, 1H), 7.01 (s, 1H), 3.76 (d, *J =* 13.8 Hz, 1H), 3.62 (d, *J =* 13.8 Hz, 1H), 3.05 (q, *J =* 6.6 Hz, 1H), 1.16 (d, *J =* 7.2 Hz, 3H).

### Examples 18 to 21: Preparation of compounds 18 to 21

Following the synthetic method of compound 1, compounds 18 to 21 were prepared by replacing the reactants with the corresponding reagents.

| Compound No. | Structural formula | **ESI-MS** (m/z) [M+H]⁺ | Substituted reagent |
|---|---|---|---|
| **18** | 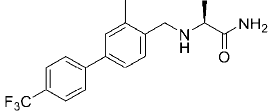 | 337.16 | 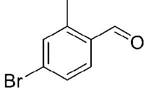 |
| **19** | 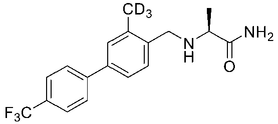 | 340.12 | 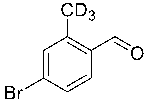 |
| **20** | 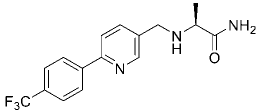 | 324.14 | 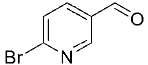 |
| **21** | 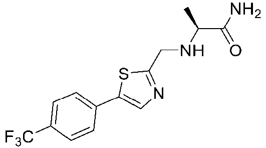 | 330.11 | 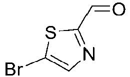 |

### Examples 22 to 24: Preparation of compounds 22 to 24

Following the preparation method for compound 3, compounds 22 to 24 were prepared by replacing the reactants with the corresponding reagents.

| Compound No. | Structural formula | **ESI-MS** (m/z) [M+H]⁺ | Substituted reagent |
|---|---|---|---|
| **22** | 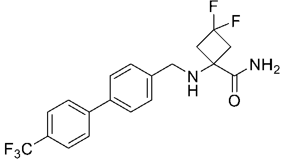 | 385.12 | 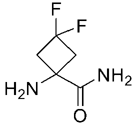 |
| **23** | 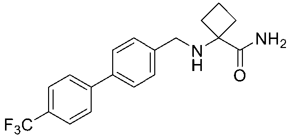 | 349.15 | 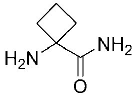 |
| **24** | 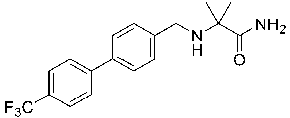 | 337.14 | 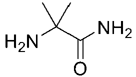 |

### Examples 25 to 27: Preparation of compounds 25 to 27

Following the preparation method for compound 4, compounds 25 to 27 were prepared by replacing the reactants with the corresponding reagents.

| Compound No. | Structural formula | **ESI-MS** (m/z) [M+H]⁺ | Substituted reagent |
|---|---|---|---|
| **25** | 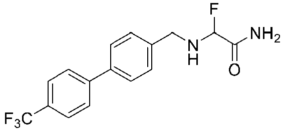 | 327.09 | 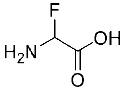 |
| **26** | 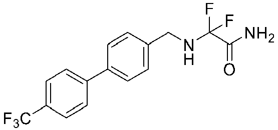 | 345.12 | 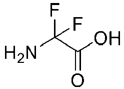 |
| **27** | 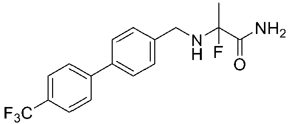 | 341.11 | 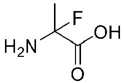 |

### Examples 28 to 37: Preparation of compounds 28 to 37

Following the preparation method for compound 3, compounds 28 to 37 were prepared by replacing the reactants with the corresponding reagents.

| Compound No. | Structural formula | **ESI-MS** (m/z) [M+H]⁺ | Substituted reagent |
|---|---|---|---|
| **28** | 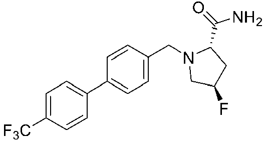 | 367.12 | 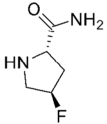 |
| **29** | 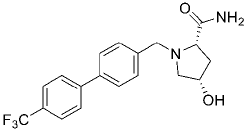 | 365.15 | 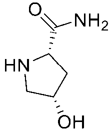 |
| **30** | 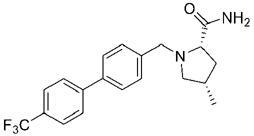 | 363.20 | 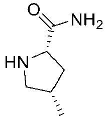 |
| **31** | 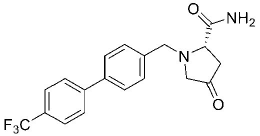 | 363.16 | 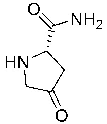 |
| **32** | 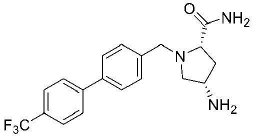 | 364.17 | 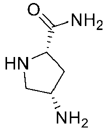 |
| **33** | 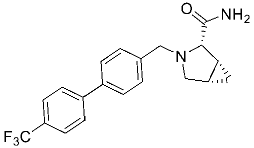 | 361.16 | 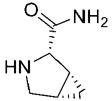 |
| **34** | 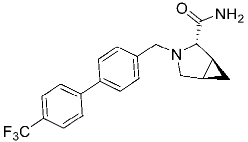 | 361.15 | 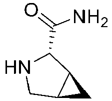 |
| **35** | 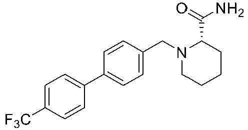 | 363.13 | 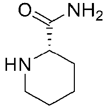 |
| **36** | 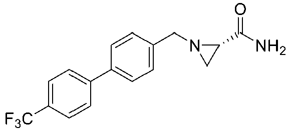 | 321.11 | 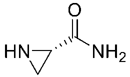 |
| **37** | 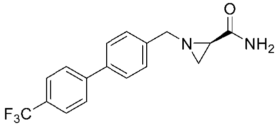 | 321.12 | 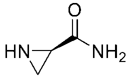 |

### Example 38: Preparation of compound 38

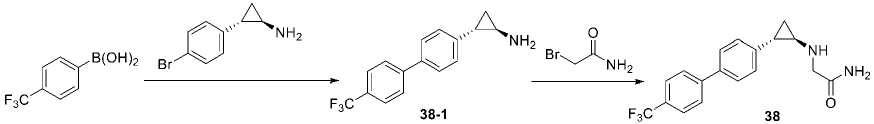

### (1) Preparation of intermediate 38-1

Following the preparation method for intermediate 1-1, 2-fluoro-4-bromobenzaldehyde was replaced with (1R, 2S)-2-(4-bromophenyl)cyclopropaneamine to give intermediate 38-1.

### (2) Preparation of compound 38

Following the preparation method for intermediate 10-1, 5-bromoisoindoline hydrochloride was substituted with Intermediate 38-1 and methyl (R)-2-bromopropanoate was replaced with 2-bromoacetamide to give compound 38. **ESI-MS** (m/z): 335.16 [M+H]⁺.

### Examples 39 to 47: Preparation of compounds 39 to 47

Following the preparation method for compound 38, compounds 39 to 47 were prepared by replacing the reactants with the corresponding reagents.

| Compound No. | Structural formula | **ESI-MS** (m/z) [M+H]⁺ | Substituted reagent |
|---|---|---|---|
| **39** | 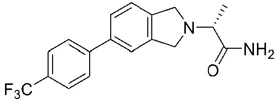 | **335.12** | 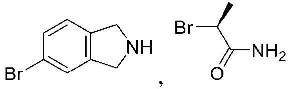 |
| **40** | 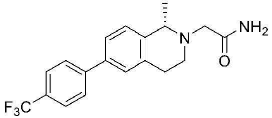 | **349.15** | 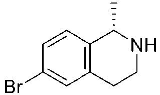 |
| **41** | 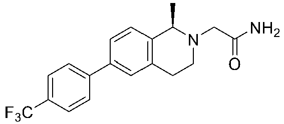 | **349.14** | 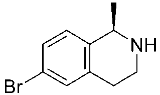 |
| **42** | 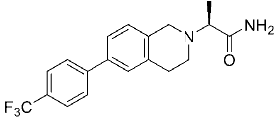 | **349.13** | 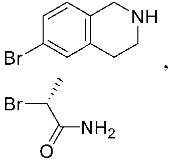 |
| **43** | 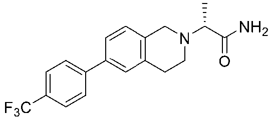 | **349.15** | 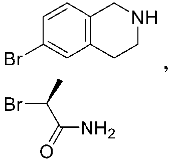 |
| **44** | 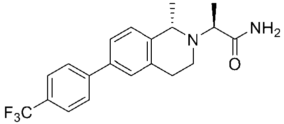 | **363.15** | 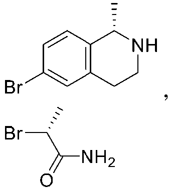 |
| **45** | 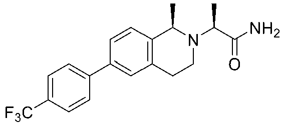 | **363.16** | 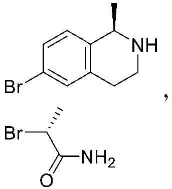 |
| **46** | 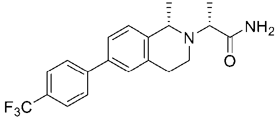 | **363.15** | 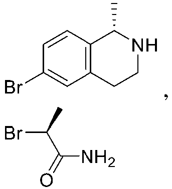 |
| **47** | 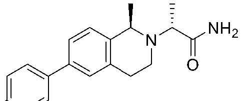 | **363.18** | 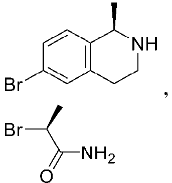 |

### Example 48: Preparation of compound 48

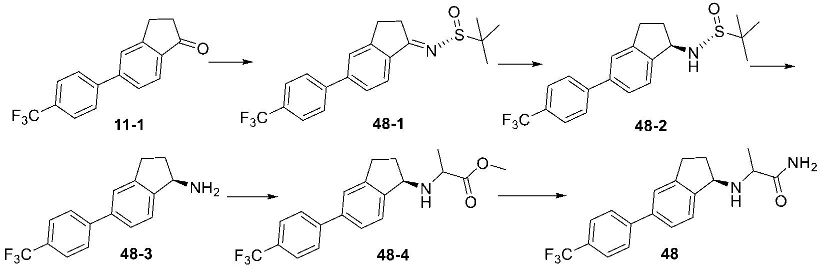

Following the preparation method for compound 11, (S)-tert-butanesulfinamide was replaced with (R)-tert-butanesulfinamide to afford compound 48. **ESI-MS** (m/z): 349.16 [M+H]⁺.

### Examples 49 to 50: Preparation of compounds 49 to 50

Following the preparation method for compound 48, compounds 49 to 50 were prepared by replacing the reactants with the corresponding reagents.

| Compound No. | Structural formula | **ESI-MS** (m/z) [M+H]⁺ | Substituted reagent |
|---|---|---|---|
| **49** | 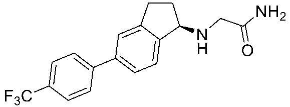 | **335.12** | 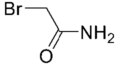 |
| **50** | 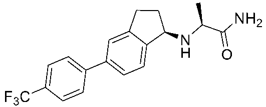 | **349.16** | 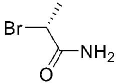 |

### Examples 51 to 52: Preparation of compounds 51 to 52

Following the preparation method for compound 11, compounds 51 to 52 were prepared by replacing the reactants with the corresponding reagents.

| Compound No. | Structural formula | **ESI-MS** (m/z) [M+H]⁺ | Substituted reagent |
|---|---|---|---|
| **51** | 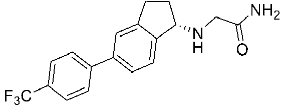 | **335.13** | 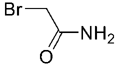 |
| **52** | 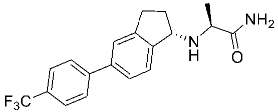 | **349.16** | 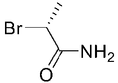 |

### Examples 53 to 63: Preparation of compounds 53 to 63

Following the preparation method for compound 16, compounds 53 to 63 were prepared by replacing the reactants with the corresponding reagents.

| Compound No. | Structural formula | **ESI-MS** (m/z) [M+H]⁺ | Substituted reagent |
|---|---|---|---|
| **53** | 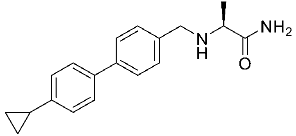 | **295.17** | 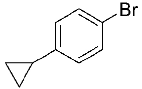 |
| **54** | 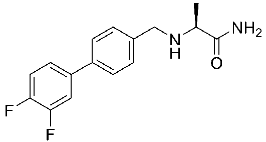 | **291.10** | 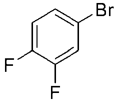 |
| **55** | 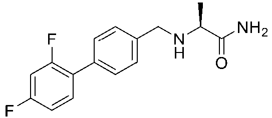 | **291.12** | 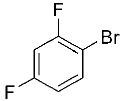 |
| **56** | 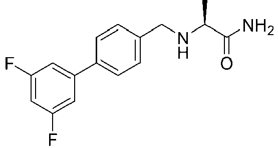 | **291.09** | 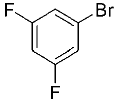 |
| **57** | 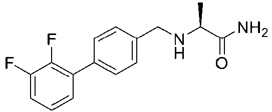 | **291.15** | 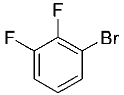 |
| **58** | 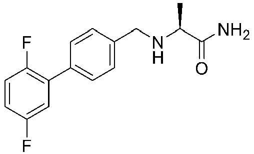 | **291.11** | 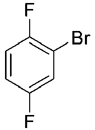 |
| **59** | 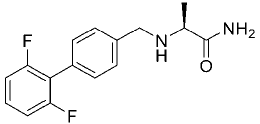 | **291.10** | 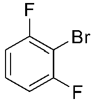 |
| **60** | 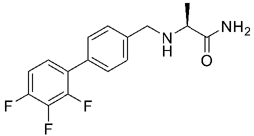 | **309.13** | 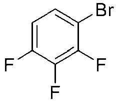 |
| **61** | 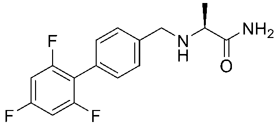 | **309.11** | 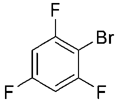 |
| **62** | 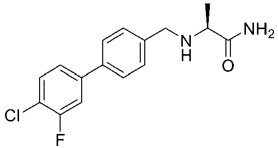 | **307.10** | 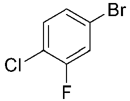 |
| **63** | 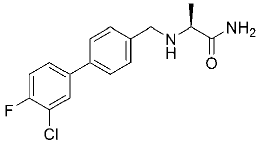 | **307.07** | 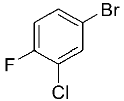 |

### Example 64: Preparation of compound 64

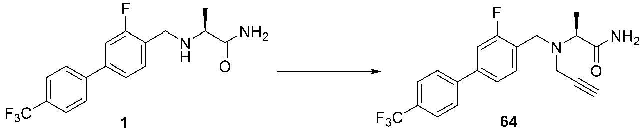

Compound 1 (0.50 mmol), 3-bromopropyne (1.5 eq), potassium carbonate (3.0 eq), and potassium iodide (0.2 eq) were dissolved in acetonitrile, and the system was allowed to react overnight at room temperature. Water (50 mL) was added, and the resulting mixture was extracted with ethyl acetate (50 mL ×3). The combined organic phases were washed with saturated sodium chloride (50 mL × 2), dried over anhydrous sodium sulfate and concentrated under reduced pressure. The residue was purified via column chromatography (dichloromethane: methanol = 50:1 to 20:1) to afford compound 64 (0.45 mmol, yield 90%). **ESI-MS** (m/z): 379.12 [M+H]⁺.

### Example 65: Preparation of methanesulfonate salt of compound 1

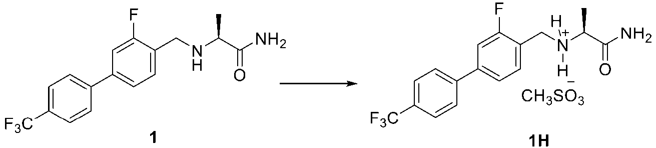

Compound 1 (420 mg, 1.23 mmol) was added to ethyl acetate (10 mL), the system was heated to 55 °C, compound 1 was completely dissolved, and then methanesulfonic acid (148 mg, 1.54 mmol) was slowly added dropwise, and the reaction was carried out at 55 °C for 2 h. The mixture was slowly cooled to room temperature, filtered, and the filter cake was washed with ethyl acetate (20 mL) to give the methanesulfonate salt of compound 1 (460 mg, yield 86%). ¹H NMR (600 MHz, DMSO-*d*₆) δ 9.23 (s, 2H), 7.97 (d, *J =* 8.4 Hz, 2H), 7.94 (s, 1H), 7.83 (d, *J =* 8.4 Hz, 2H), 7.75-7.69 (m, 3H), 7.66 (s, 1H), 4.22-4.16 (m, 2H), 3.87 (q, *J =* 6.6 Hz, 1H), 2.29 (s, 3H), 1.44 (d, *J =* 6.6 Hz, 3H).

### Control compounds for test examples

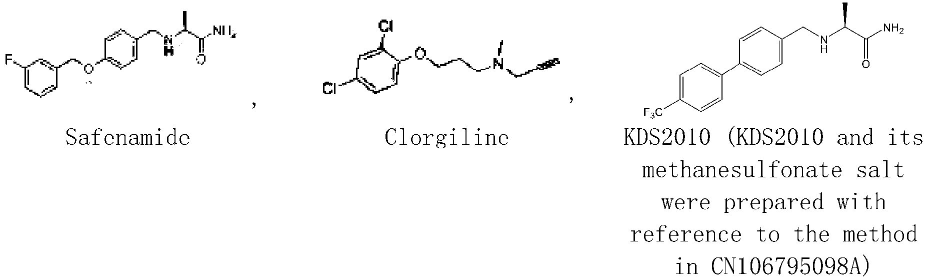

### Test Example 1: Determination of inhibitory activity of compounds against MAO-B

### 1.1 Test materials

| Reagent Name | Manufacturer | Cat. No. |
|---|---|---|
| MAO-B enzyme | Acro Biosystems | MAB-H5547 |
| Amplex^{™} Red Monoamine Oxidase Assay Kit | Thermo | A12214 |

### 1.2 Test method

(1) Sample preparation: The compound at 10 mM was first diluted 100-fold to obtain a high-concentration solution of 0.1 mM (100000 nM). This solution was serially diluted 5-fold to afford 20000 nM, 4000 nM, 800 nM, 160 nM, 32 nM, 6.4 nM and 1.28 nM, generating 8 concentrations in total. 0.05 M sodium phosphate buffer (pH 7.4) was prepared meanwhile.
(2) Human monoamine oxidase B at 290 µg/mL was diluted to 1/121 with 0.05 M sodium phosphate buffer. Enzyme buffers were prepared by mixing 10 µL of each of 8-concentration compound solutions with the diluted enzyme solution to a final volume of 100 µL. The enzyme buffer was dispensed into a 96-well plate and incubated for 30 min.
(3) Working buffer was formulated by combining 20 mM Amplex Red (200 µL), 100 mM benzylamine substrate (200 µL) and 200 U/mL horseradish peroxidase (100 µL) into 0.05 M sodium phosphate buffer (pH 7.4) (9.5 mL). 100 µL of the working buffer was mixed with the enzyme buffer in (2) at a 1:1 ratio and incubated for 30 min. Fluorescence signals were subsequently measured with excitation wavelength set at 530-560 nm and emission wavelength at 590 nm.
(4) The enzyme activity inhibition rate data were processed using formula (1) to calculate the inhibition rate corresponding to different concentrations of compounds. The RLU compound is the RLU value detected after compound incubation, the RLU blank well is the RLU detected without monoamine oxidase B, and the RLU vehicle control well is the RLU detected with 0.05 M sodium phosphate buffer. The inhibition rate curve was plotted and the IC₅₀ value was calculated using GraphPad Prism software. 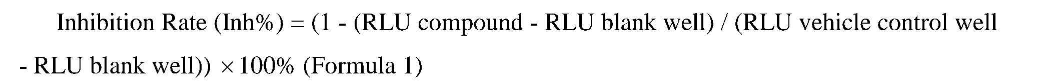 Inhibition Rate (Inh%) = (1 - (RLU compound - RLU blank well) / (RLU vehicle control well - RLU blank well)) × 100% (Formula 1)

The representative compounds in the above test examples were tested according to the above methods, and it was found that they exhibited potent inhibitory activity against MAO-B. The activity data of exemplary compounds are shown in the table below.

| Compound | MAO-B |
|---|---|
| | IC₅₀ (nM) |
| 1 | A |
| 2 | B |
| 4 | B |
| 5 | B |
| 6 | A |
| 10 | A |
| 12 | B |
| Safenamide | A |
| KDS2010 | B |

| | |
|---|---|
| A: IC₅₀<50 nM, B: 50 nM≤ IC₅₀<100 nM, C: 100 nM≤ IC₅₀<1000 nM, D: 1000 nM≤ IC₅₀<2000 nM, E: 2000 nM≤ IC₅₀<10000 nM, F: IC₅₀≥10000 nM | |

### Test Example 2: Determination of inhibitory activity of compounds against MAO-A

### 2.1 Test materials

| Reagent Name | Manufacturer | Cat. No. |
|---|---|---|
| MAO-A enzyme | Acro Biosystems | MAA-H5547 |
| MAO-Glo^{™} Assay | Promega | OF1R3N8231017 |

### 2.2 Test method

(1) Sample preparation: The 10 mM compound was first diluted 50-fold, and then the high concentration of 0.2 mM (200000 nM) was serially diluted 5-fold to afford 3 concentrations of 40000 nM, 8000 nM and 1600 nM. The clorgiline was prepared into 8 concentrations of 40000 nM, 8000 nM, 1600 nM, 320 nM, 64 nM, 12.8 nM, 2.56 nM and 0.5 nM. MAO Reaction Buffer was prepared meanwhile.
(2) 270 µg/mL human monoamine oxidase A was diluted to 4 µg/mL with MAO Reaction Buffer and 25 µL of the resultant enzyme solution was dispensed into each well of a 96-well plate.
(3) The prepared compounds of different concentrations were added to the corresponding 96-well plate at 12.5 µL/well. After brief mixing, the plate was incubated for 30 min.
(4) 4 mM MAO Substrate was diluted to 160 µM with MAO Reaction Buffer and added to a 96-well plate at 12.5 µL/well, and incubation was continued for another 30 min.
(5) 50 µL of Reconstituted Luciferin Detection Reagent was added to each well. The system was briefly mixed and incubated in the dark for 20 min prior to luminescence measurement.
(6) The enzyme activity inhibition rate data were processed using formula (1) to calculate the inhibition rate corresponding to different concentrations of compounds. The RLU compound is the RLU value detected after compound incubation, the RLU blank well is the RLU detected without monoamine oxidase A, and the RLU vehicle control well is the RLU detected with MAO Reaction Buffer.

The representative compounds in the above test examples were tested according to the above methods, and it was found that they exhibited very weak inhibitory activity against MAO-A. The activity data of exemplary compounds are shown in the table below.

| Compound | MAO-A |
|---|---|
| | IC₅₀ (nM) |
| 1 | F |
| 2 | E |
| 6 | F |
| 10 | F |
| Safenamide | F |
| KDS2010 | F |
| Clorgiline | A |

| | |
|---|---|
| A: IC₅₀<50 nM, B: 50 nM≤IC₅₀<100 nM, C: 100 nM≤ IC₅₀<1000 nM, D: 1000 nM≤ IC₅₀ < 2000 nM, E: 2000 nM≤ IC₅₀ < 10000 nM, F: IC₅₀≥10000 nM | |

Experimental results demonstrate that the compounds of the present invention possess selective inhibitory activity against MAO-B.

### Test Example 3: Test of weight-lowering efficacy of compounds in DIO mice

### 3.1 Experimental animals

DIO mice, SPF grade, male, 45-55 g, purchased from GemPharmatech Co., Ltd., Jiangsu.

### 3.2 Experimental grouping and procedure

The DIO mouse model was established by feeding C57BL/6J mice with a high-fat diet for several weeks to induce obesity. After one-week adaptive acclimatization with stabilized weight, animals were randomized into groups by weight uniformity (d0), with 6 mice per group. The vehicle group (purified water) and administration group were given intragastric administration once daily, with the dosage of 10 mg/kg for the administration group. Body weight of mice and food consumption were measured daily, and relevant data were recorded.

Formula for calculating weight change: Weight change = Wt - WO, where Wt is the average daily weight of the mouse and WO is the average weight of the mouse on day 0.

Formula for calculating weight gain rate: Weight gain rate = (Wt-WO)/WO*100%, where Wt is the average daily weight of the mouse and WO is the average weight of the mouse on day 0.

Formula for calculating food intake: Food intake = F0 - Ft, where F0 is the weight of the basic food and Ft is the weight of the remaining food.

### 3.3 Experimental results

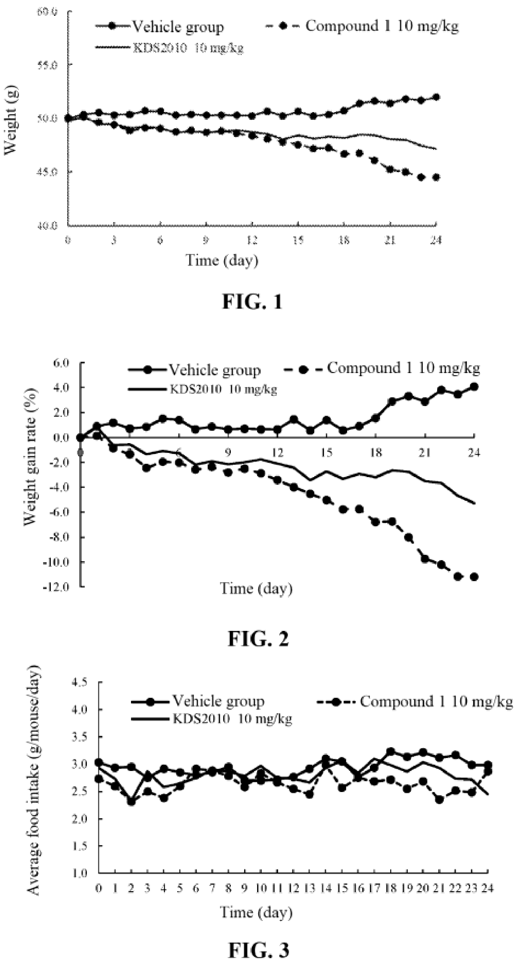

The experimental results show that the compound of the present invention can significantly reduce the body weight of mice without affecting food intake, and is even superior to the positive control drug KDS2010. Exemplary experimental results are shown in the table below and in FIG. 1, FIG. 2 and FIG. 3.

| Days | Mouse weight (g) | | | Mouse weight change (g) | | | Mouse weight change rate (%) | | |
|---|---|---|---|---|---|---|---|---|---|
| | Vehicle group | Compound 1 group | KDS2 010 group | Vehicle group | Compound 1 group | KDS20 10 group | Vehicle group | Compound 1 group | KDS20 10 group |
| 0 | 50.0 | 50.1 | 49.7 | 0 | 0 | 0 | 0 | 0 | 0 |
| 1 | 50.4 | 50.1 | 50.1 | 0.4 | 0 | 0.4 | 0.8 | 0 | 0.8 |
| 2 | 50.6 | 49.6 | 49.4 | 0.6 | -0.5 | -0.3 | 1.2 | -1 | -0.6 |
| 3 | 50.3 | 49.4 | 49.5 | 0.3 | -0.7 | -0.2 | 0.6 | -1.4 | -0.4 |
| 4 | 50.4 | 49.8 | 49.1 | 0.4 | -0.3 | -0.6 | 0.8 | -0.6 | -1.2 |
| 5 | 50.7 | 49.1 | 49.2 | 0.7 | -1.0 | -0.5 | 1.4 | -2 | -1 |
| 6 | 50.7 | 49.1 | 49.1 | 0.7 | -1.0 | -0.6 | 1.4 | -2 | -1.2 |
| 7 | 50.3 | 48.8 | 48.7 | 0.3 | -1.3 | -1.0 | 0.6 | -2.6 | -2 |
| 8 | 50.4 | 48.9 | 48.8 | 0.4 | -1.2 | -0.9 | 0.8 | -2.4 | -1.8 |
| 9 | 50.3 | 48.7 | 48.7 | 0.3 | -1.4 | -1.0 | 0.6 | -2.8 | -2 |
| 10 | 50.3 | 48.8 | 48.8 | 0.3 | -1.3 | -0.9 | 0.6 | -2.6 | -1.8 |
| 11 | 50.3 | 48.6 | 48.9 | 0.3 | -1.5 | -0.8 | 0.6 | -3 | -1.6 |
| 12 | 50.3 | 48.4 | 48.7 | 0.3 | -1.7 | -1.0 | 0.6 | -3.4 | -2 |
| 13 | 50.7 | 48.1 | 48.4 | 0.7 | -2.0 | -1.3 | 1.4 | -4 | -2.6 |
| 14 | 50.2 | 47.8 | 48.1 | 0.2 | -2.3 | -1.6 | 0.4 | -4.6 | -3.2 |
| 15 | 50.7 | 47.6 | 48.4 | 0.7 | -2.5 | -1.3 | 1.4 | -5 | -2.6 |
| 16 | 50.2 | 47.2 | 48.1 | 0.2 | -2.9 | -1.6 | 0.4 | -5.8 | -3.2 |
| 17 | 50.4 | 47.2 | 48.3 | 0.4 | -2.9 | -1.4 | 0.8 | -5.8 | -2.8 |
| 18 | 50.7 | 46.7 | 48.3 | 0.7 | -3.4 | -1.4 | 1.4 | -6.8 | -2.8 |
| 19 | 51.4 | 46.7 | 48.5 | 1.4 | -3.4 | -1.2 | 2.8 | -6.8 | -2.4 |
| 20 | 51.6 | 46.1 | 48.4 | 1.6 | -4.0 | -1.3 | 3.2 | -8 | -2.6 |
| 21 | 51.4 | 45.2 | 48.0 | 1.4 | -4.9 | -1.7 | 2.8 | -9.8 | -3.4 |
| 22 | 51.9 | 45.0 | 48.0 | 1.9 | -5.1 | -1.7 | 3.8 | -10.2 | -3.4 |
| 23 | 51.7 | 44.5 | 47.5 | 1.7 | -5.6 | -2.2 | 3.4 | -11.2 | -4.4 |
| 24 | 52.0 | 44.5 | 47.2 | 2 | -5.6 | -2.5 | 4 | -11.2 | -5 |

| | | | | | | | | | |
|---|---|---|---|---|---|---|---|---|---|
| Note: Compound 1 and KDS2010 used in Test Example 3 are in methanesulfonate salt form, while the compounds used in other test examples are in free form. | | | | | | | | | |

## Claims

1. A compound as shown in formula (I), a tautomer, stereoisomer or pharmaceutically acceptable salt thereof, having the following structure 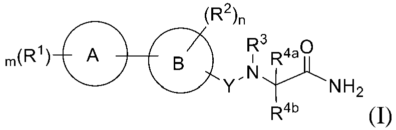 wherein
rings A and B are each independently selected from C₆₋₁₀ aryl and 5- to 10-membered heteroaryl;
R¹ and R² are each independently selected from deuterium, halogen, cyano, nitro, amino, hydroxy, mercapto, -SF₅, -COOH, -OR⁵, -SR⁵, -NR⁵R⁵, -C(O)R⁵, -C(O)OR⁵, -C(O)NR⁵R⁵, - OC(O)R⁵, -S(O)₁₋₂R⁵, -S(O)₂NR⁵R⁵, -N(R⁵)S(O)₂R⁵, -N(R⁵)C(O)R⁵, -N(R⁵)C(O)NR⁵, C₁₋₆ alkyl, C₂₋₆ alkenyl, C₂₋₆ alkynyl, C₃₋₁₀ cycloalkyl, C₃₋₁₀ cycloalkenyl, C₆₋₁₀ aryl, 3- to 10-membered heterocyclyl and 5- to 10-membered heteroaryl; wherein the C₁₋₆ alkyl, C₂₋₆ alkenyl, C₂₋₆ alkynyl, C₃₋₁₀ cycloalkyl, C₃₋₁₀ cycloalkenyl, C₆₋₁₀ aryl, 3- to 10-membered heterocyclyl and 5- to 10-membered heteroaryl are each optionally substituted with 0 to 6 identical or different substituents selected from deuterium, cyano, amino, hydroxyl and halogen;
each R⁵ is independently selected from hydrogen, deuterium, C₁₋₆ alkyl, C₂₋₆ alkenyl, C₂₋₆ alkynyl, C₃₋₁₀ cycloalkyl and 3- to 10-membered heterocyclyl, wherein the C₁₋₆ alkyl, C₂₋₆ alkenyl, C₂₋₆ alkynyl, C₃₋₁₀ cycloalkyl and 3- to 10-membered heterocyclyl are each optionally substituted with 0 to 6 identical or different substituents selected from deuterium, cyano, amino, hydroxyl and halogen;
m and n are each independently selected from 0 to 6;
Y is independently selected from (CR⁶R⁷)₁₋₂ or is a single bond;
R⁶ and R⁷ are each independently selected from hydrogen, deuterium, halogen, C₁₋₆ alkyl, C₁₋₆ haloalkyl, C₁₋₆ alkoxy, C₁₋₆ haloalkoxy, and hydroxy-substituted C₁₋₆ alkyl;
alternatively, when Y is selected from (CR⁶R⁷)₂, one R⁶ or R⁷, together with two carbon atoms, forms C₃₋₆ cycloalkyl or 3- to 6-membered heterocyclyl; the remaining R⁶ and R⁷ are selected from hydrogen, deuterium and halogen; wherein the heterocyclyl contains 0 to 2 heteroatoms selected from N, O, C(=O) and S, and the numbers of the heteroatoms are not 0 simultaneously; the C₃₋₆ cycloalkyl and 3- to 6-membered heterocyclyl are each optionally substituted with 0 to 6 identical or different substituents selected from deuterium, C₁₋₆ alkyl, cyano, amino, hydroxyl and halogen;
alternatively, R² and R⁶, together with the atoms to which they are attached, form C₅₋₈ carbocyclyl or 5- to 8-membered heterocyclyl; wherein the heterocyclyl contains 0 to 2 heteroatoms selected from N, O, C(=O) and S, and the numbers of the heteroatoms are not 0 simultaneously; the C₅₋₈ carbocyclyl and 5- to 8-membered heterocyclyl are each optionally substituted with 0 to 6 identical or different substituents selected from deuterium, cyano, amino, hydroxy, C₁₋₆ alkyl and halogen; R⁷ is selected from hydrogen, deuterium, halogen, C₁₋₆ alkyl, C₁₋₆ haloalkyl, C₁₋₆ alkoxy, C₁₋₆ haloalkoxy and hydroxy-substituted C₁₋₆ alkyl;
R³ is independently selected from hydrogen, deuterium, C₁₋₆ alkyl, C₂₋₆ alkenyl, C₂₋₆ alkynyl, C₁₋₆ haloalkyl, C₁₋₆ alkoxy, C₁₋₆ haloalkoxy, hydroxy-substituted C₁₋₆ alkyl and C₃₋₈ cycloalkyl;
alternatively, R² and R³, together with the atoms to which they are attached, form 4- to 8-membered nitrogen-containing heterocyclyl; wherein the 4- to 8-membered nitrogen-containing heterocyclyl contains 1 to 2 N heteroatoms and 0 to 2 heteroatoms selected from O, C(=O) and S; the 4- to 8-membered nitrogen-containing heterocyclyl is optionally substituted with 0 to 6 identical or different substituents selected from deuterium, C₁₋₆ alkyl, cyano, amino, hydroxyl and halogen;
R^{4a} and R^{4b} are each independently selected from hydrogen, deuterium, halogen, hydroxy, cyano, amino, mercapto, C₁₋₆ alkyl, C₁₋₆ alkoxy, C₂₋₆ alkenyl, C₂₋₆ alkynyl, C₃₋₁₀ cycloalkyl and C₃₋₁₀ cycloalkenyl; the C₁₋₆ alkyl, C₁₋₆ alkoxy, C₂₋₆ alkenyl, C₂₋₆ alkynyl, C₃₋₁₀ cycloalkyl or C₃₋₁₀ cycloalkenyl is optionally substituted with 0 to 3 substituents selected from deuterium, halogen, hydroxy, amino, nitro, cyano, mercapto, C₁₋₆ alkyl and C₁₋₆ alkoxy;
alternatively, R^{4a} and R^{4b}, together with the carbon atoms to which they are attached, form C₃₋₆ carbocyclyl or 3- to 6-membered heterocyclyl; wherein the heterocyclyl contains 0 to 2 heteroatoms selected from N, O, C(=O) and S, and the numbers of the heteroatoms are not 0 simultaneously; the C₃₋₆ carbocyclyl and 3- to 6-membered heterocyclyl are each optionally substituted with 0 to 6 identical or different substituents selected from deuterium, cyano, amino, hydroxy, halogen and C₁₋₆ alkyl;
alternatively, R³ and R^{4a}, together with the atoms to which they are attached, form 3- to 8-membered nitrogen-containing heterocyclyl; wherein the nitrogen-containing heterocyclyl contains 1 to 3 N heteroatoms and 0 to 2 heteroatoms selected from O, C(=O) and S; the 3- to 8-membered nitrogen-containing heterocyclyl is optionally substituted with 0 to 6 identical or different substituents selected from deuterium, cyano, amino, hydroxy, C₁₋₃ alkyl, C₁₋₃ alkoxy, C₁₋₃ haloalkyl, C₁₋₃ haloalkoxy and halogen; R^{4b} is selected from hydrogen, deuterium, halogen, hydroxy, cyano, amino, mercapto, C₁₋₆ alkyl, C₁₋₆ alkoxy, C₂₋₆ alkenyl, C₂₋₆ alkynyl, C₃₋₁₀ cycloalkyl and C₃₋₁₀ cycloalkenyl; the C₁₋₆ alkyl, C₁₋₆ alkoxy, C₂₋₆ alkenyl, C₂₋₆ alkynyl, C₃₋₁₀ cycloalkyl or C₃₋₁₀ cycloalkenyl is optionally substituted with 0 to 3 substituents selected from deuterium, halogen, hydroxy, amino, nitro, cyano, mercapto, C₁₋₆ alkyl and C₁₋₆ alkoxy,
the compound of formula (I) does not include the following compounds:

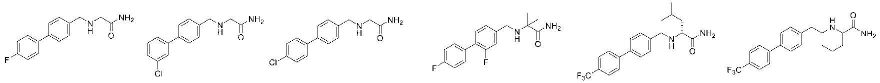

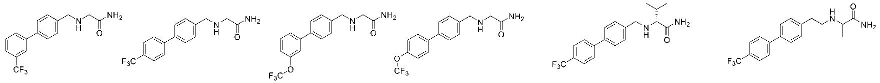

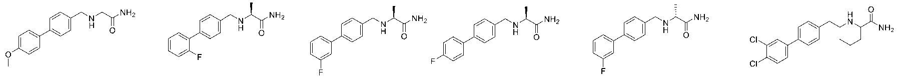

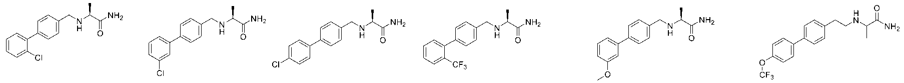

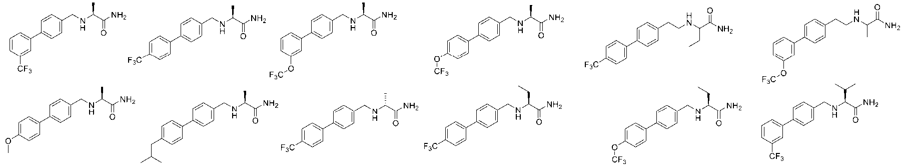

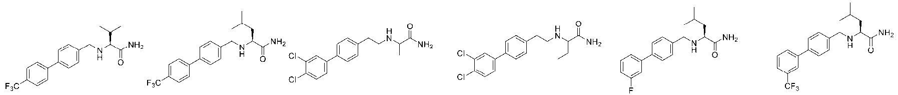

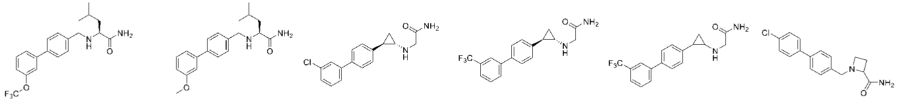

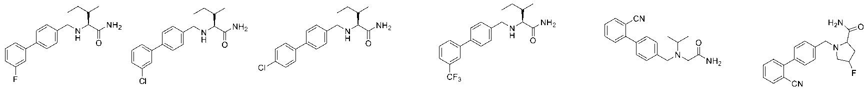

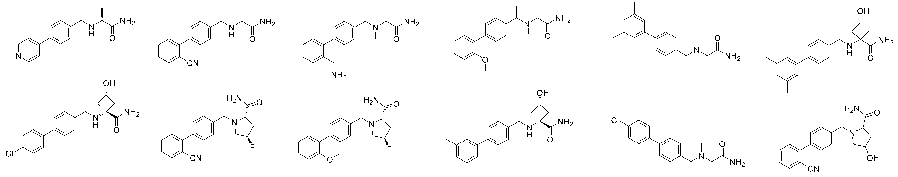

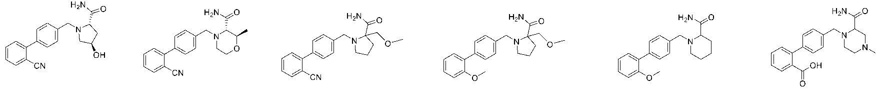

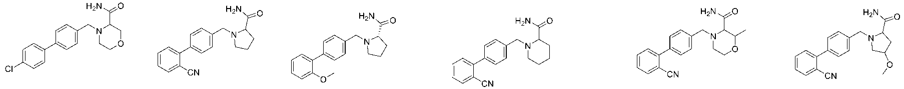

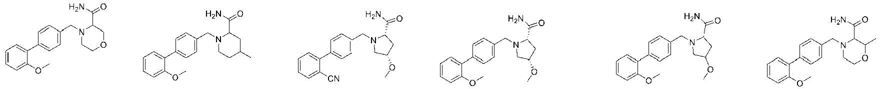

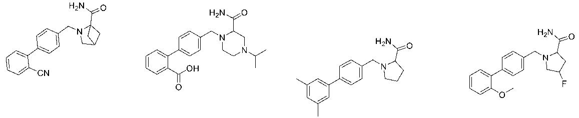

2. The compound according to claim 1, a tautomer, stereoisomer or pharmaceutically acceptable salt thereof, wherein rings A and B are each independently selected from phenyl, pyridinyl, pyrimidinyl, pyrazinyl, pyridazinyl, thienyl, furyl, pyrrolyl, thiazolyl, oxazolyl, pyrazolyl and imidazolyl;
preferably, rings A and B are each independently selected from phenyl, pyridinyl, thienyl, furyl, pyrrolyl, thiazolyl, oxazolyl, pyrazolyl and imidazolyl;
more preferably, rings A and B are each independently selected from phenyl, pyridinyl, thienyl and thiazolyl;
still more preferably, rings A and B are each independently selected from phenyl.

3. The compound according to any one of claims 1 to 2, a tautomer, stereoisomer or pharmaceutically acceptable salt thereof, wherein R¹ and R² are each independently selected from deuterium, halogen, -SF₅, -OR⁵, -SR⁵, -NR⁵R⁵, -C(O)R⁵, -C(O)OR⁵, -C(O)NR⁵R⁵, -OC(O)R⁵, - S(O)₁₋₂R⁵, -S(O)₂NR⁵R⁵, -N(R⁵)S(O)₂R⁵, -N(R⁵)C(O)R⁵, -N(R⁵)C(O)NR⁵, C₁₋₆ alkyl, C₂₋₆ alkenyl, C₂₋₆ alkynyl, C₃₋₁₀ cycloalkyl, C₃₋₁₀ cycloalkenyl, C₆₋₁₀ aryl, 3- to 10-membered heterocyclyl and 5- to 10-membered heteroaryl; wherein the C₁₋₆ alkyl, C₂₋₆ alkenyl, C₂₋₆ alkynyl, C₃₋₁₀ cycloalkyl, C₃₋₁₀ cycloalkenyl, C₆₋₁₀ aryl, 3- to 10-membered heterocyclyl and 5- to 10-membered heteroaryl are each optionally substituted with 0 to 6 identical or different substituents selected from deuterium, cyano, amino, hydroxyl and halogen;
each R⁵ is independently selected from hydrogen, deuterium, C₁₋₆ alkyl, C₂₋₆ alkenyl, C₂₋₆ alkynyl, C₃₋₁₀ cycloalkyl and 3- to 10-membered heterocyclyl, wherein the C₁₋₆ alkyl, C₂₋₆ alkenyl, C₂₋₆ alkynyl, C₃₋₁₀ cycloalkyl and 3- to 10-membered heterocyclyl are each optionally substituted with 0 to 6 identical or different substituents selected from deuterium, cyano, amino, hydroxyl and halogen;
alternatively,
R¹ and R² are each independently selected from deuterium, halogen, -SF₅, -OR⁵, -SR⁵, -NR⁵R⁵, -C(O)R⁵, -C(O)OR⁵, -C(O)NR⁵R⁵, -OC(O)R⁵, -S(O)₁₋₂R⁵, -S(O)₂NR⁵R⁵, -N(R⁵)S(O)₂R⁵, - N(R⁵)C(O)R⁵, -N(R⁵)C(O)NR⁵, C₁₋₆ alkyl, C₃₋₁₀ cycloalkyl and 3- to 10-membered heterocyclyl; wherein the C₁₋₆ alkyl, C₃₋₁₀ cycloalkyl and 3- to 10-membered heterocyclyl are each optionally substituted with 0 to 6 identical or different substituents selected from deuterium, cyano, amino, hydroxyl and halogen;
each R⁵ is independently selected from hydrogen, deuterium, C₁₋₆ alkyl, C₃₋₁₀ cycloalkyl and 3- to 10-membered heterocyclyl, wherein the C₁₋₆ alkyl, C₃₋₁₀ cycloalkyl and 3- to 10- membered heterocyclyl are each optionally substituted with 0 to 6 identical or different substituents selected from deuterium, cyano, amino, hydroxyl and halogen;
alternatively,
R¹ and R² are each independently selected from deuterium, halogen, -SF₅, -OR⁵, -SR⁵, -NR⁵R⁵, -C(O)R⁵, -S(O)₁₋₂R⁵, C₁₋₆ alkyl, C₃₋₁₀ cycloalkyl and 3- to 10-membered heterocyclyl; wherein the C₁₋₆ alkyl, C₃₋₁₀ cycloalkyl and 3- to 10-membered heterocyclyl are each optionally substituted with 0 to 6 identical or different substituents selected from deuterium, cyano, amino, hydroxyl and halogen;
each R⁵ is independently selected from hydrogen, deuterium, C₁₋₆ alkyl, C₃₋₁₀ cycloalkyl and 3- to 10-membered heterocyclyl, wherein the C₁₋₆ alkyl, C₃₋₁₀ cycloalkyl and 3- to 10- membered heterocyclyl are each optionally substituted with 0 to 6 identical or different substituents selected from deuterium, cyano, amino, hydroxyl and halogen;
alternatively,
R¹ and R² are each independently selected from deuterium, halogen, -SF₅, -OR⁵, -SR⁵, C₁₋₆ alkyl, C₃₋₁₀ cycloalkyl and 3- to 10-membered heterocyclyl; wherein the C₁₋₆ alkyl, C₃₋₁₀ cycloalkyl and 3- to 10-membered heterocyclyl are each optionally substituted with 0 to 6 identical or different substituents selected from deuterium, cyano, amino, hydroxyl and halogen;
each R⁵ is independently selected from hydrogen, deuterium, C₁₋₆ alkyl, C₃₋₁₀ cycloalkyl and 3- to 10-membered heterocyclyl, wherein the C₁₋₆ alkyl, C₃₋₁₀ cycloalkyl and 3- to 10- membered heterocyclyl are each optionally substituted with 0 to 6 identical or different substituents selected from deuterium, cyano, amino, hydroxyl and halogen;
alternatively,
R¹ and R² are each independently selected from deuterium, halogen, -SF₅, -OR⁵, -SR⁵, C₁₋₆ alkyl, C₃₋₁₀ cycloalkyl and 3- to 10-membered heterocyclyl; wherein the C₁₋₆ alkyl, C₃₋₁₀ cycloalkyl and 3- to 10-membered heterocyclyl are each optionally substituted with 0 to 6 identical or different substituents selected from deuterium and halogen;
each R⁵ is independently selected from hydrogen, deuterium, C₁₋₆ alkyl, C₃₋₁₀ cycloalkyl and 3- to 10-membered heterocyclyl, wherein the C₁₋₆ alkyl, C₃₋₁₀ cycloalkyl and 3- to 10- membered heterocyclyl are each optionally substituted with 0 to 6 identical or different substituents selected from deuterium and halogen;
alternatively,
R¹ and R² are each independently selected from deuterium, halogen, -SF₅, -OR⁵, -SR⁵, C₁₋₆ alkyl, C₃₋₆ cycloalkyl and 3- to 6-membered heterocyclyl; wherein the C₁₋₆ alkyl, C₃₋₆ cycloalkyl and 3- to 6-membered heterocyclyl are each optionally substituted with 0 to 6 identical or different substituents selected from deuterium and halogen;
each R⁵ is independently selected from hydrogen, deuterium, C₁₋₆ alkyl, C₃₋₆ cycloalkyl and 3-to 6-membered heterocyclyl, wherein the C₁₋₆ alkyl, C₃₋₆ cycloalkyl and 3- to 6- membered heterocyclyl are each optionally substituted with 0 to 6 identical or different substituents selected from deuterium and halogen;
alternatively,
R¹ and R² are each independently selected from deuterium, fluorine, chlorine, bromine, -SF₅, - OR⁵, -SR⁵, C₁₋₆ alkyl, C₃₋₆ cycloalkyl and 3- to 6-membered heterocyclyl; wherein the C₁₋₆ alkyl, C₃₋₆ cycloalkyl and 3- to 6-membered heterocyclyl are each optionally substituted with 0 to 6 identical or different substituents selected from deuterium, fluorine, chlorine and bromine;
each R⁵ is independently selected from C₁₋₆ alkyl, C₃₋₆ cycloalkyl and 3- to 6-membered heterocyclyl, wherein the C₁₋₆ alkyl, C₃₋₆ cycloalkyl and 3- to 6- membered heterocyclyl are each optionally substituted with 0 to 6 identical or different substituents selected from deuterium, fluorine, chlorine and bromine;
alternatively,
R¹ and R² are each independently selected from fluorine, chlorine, -SF₅, -SCF₃, methyl, ethyl, propyl, isopropyl, cyclopropyl, butyl, isobutyl, tert-butyl, cyclobutyl, methoxy, ethoxy, propoxy, isopropoxy, cyclopropoxy, butoxy, isobutoxy, tert-butoxy and cyclobutoxy; wherein methyl, ethyl, propyl, isopropyl, cyclopropyl, butyl, isobutyl, tert-butyl, cyclobutyl, methoxy, ethoxy, propoxy, isopropoxy, cyclopropoxy, butoxy, isobutoxy, tert-butoxy and cyclobutoxy are each optionally substituted with 0 to 6 identical or different substituents selected from deuterium, fluorine, chlorine and bromine;
alternatively,
R¹ and R² are each independently selected from fluorine, chlorine, -SF₅, -SCF₃, -OCF₃, -CF₃, methyl, deuterated methyl and cyclopropyl;
alternatively, R¹ is selected from -CF₃, and R² is selected from fluorine.

4. The compound according to any one of claims 1 to 3, a tautomer, stereoisomer or pharmaceutically acceptable salt thereof, wherein m and n are each independently selected from 0 to 5, preferably 0, 1, 2, 3, 4, and 5, more preferably 0, 1, 2 and 3;
alternatively, m is selected from 0 to 3, preferably 1, 2 and 3, more preferably 1; n is selected from 0 and 1.

5. The compound according to any one of claims 1 to 4, a tautomer, stereoisomer or pharmaceutically acceptable salt thereof, wherein Y is independently selected from (CR⁶R⁷)₁₋₂;
R⁶ and R⁷ are each independently selected from hydrogen, deuterium, halogen, C₁₋₆ alkyl, C₁₋₆ haloalkyl, C₁₋₆ alkoxy, C₁₋₆ haloalkoxy, and hydroxy-substituted C₁₋₆ alkyl;
alternatively, when Y is selected from (CR⁶R⁷)₂, one R⁶ or R⁷, together with two carbon atoms, forms C₃₋₆ cycloalkyl or 3- to 6-membered heterocyclyl; the remaining R⁶ and R⁷ are selected from hydrogen, deuterium and halogen; wherein the heterocyclyl contains 0 to 2 heteroatoms selected from N, O, C(=O) and S, and the numbers of the heteroatoms are not 0 simultaneously; the C₃₋₆ cycloalkyl and 3- to 6-membered heterocyclyl are each optionally substituted with 0 to 6 identical or different substituents selected from deuterium, cyano, amino, hydroxyl and halogen;
alternatively, R² and R⁶, together with the atoms to which they are attached, form C₅₋₈ carbocyclyl or 5- to 8-membered heterocyclyl; wherein the heterocyclyl contains 0 to 2 heteroatoms selected from N, O, C(=O) and S, and the numbers of the heteroatoms are not 0 simultaneously; the C₅₋₈ carbocyclyl and 5- to 8-membered heterocyclyl are each optionally substituted with 0 to 6 identical or different substituents selected from deuterium, cyano, amino, hydroxy, and halogen; R⁷ is selected from hydrogen, deuterium, halogen, C₁₋₆ alkyl, C₁₋₆ haloalkyl, C₁₋₆ alkoxy, C₁₋₆ haloalkoxy and hydroxy-substituted C₁₋₆ alkyl;
preferably,
Y is independently selected from (CR⁶R⁷)₁₋₂;
R⁶ and R⁷ are each independently selected from hydrogen, deuterium, methyl and halogen; alternatively, R⁶ and R⁷, together with the carbon atoms to which they are attached, form 3-membered cycloalkyl; wherein the 3-membered cycloalkyl is optionally substituted with 0 to 4 identical or different substituents selected from deuterium, cyano, amino, hydroxyl and halogen;
alternatively, R² and R⁶, together with the atoms to which they are attached, form C₅₋₆ carbocyclyl or 5- to 6-membered heterocyclyl; wherein the heterocyclyl contains 0 to 2 heteroatoms selected from N, O, C(=O) and S, and the numbers of the heteroatoms are not 0 simultaneously; the C₅₋₆ carbocyclyl and 5- to 6-membered heterocyclyl are each optionally substituted with 0 to 6 identical or different substituents selected from deuterium and halogen; R⁷ is selected from hydrogen, deuterium, methyl and halogen;
more preferably,
Y is independently selected from (CR⁶R⁷)₁₋₂;
R⁶ and R⁷ are each independently selected from hydrogen and methyl;
alternatively, when Y is selected from (CR⁶R⁷)₂, one R⁶ or R⁷, together with two carbon atoms, forms 3-membered cycloalkyl;
alternatively, R² and R⁶, together with the atoms to which they are attached, form 5-membered carbocyclyl; R⁷ is selected from hydrogen and methyl.

6. The compound according to any one of claims 1 to 5, a tautomer, stereoisomer or pharmaceutically acceptable salt thereof, wherein R³ is independently selected from hydrogen, deuterium, C₁₋₆ alkyl, C₂₋₆ alkynyl and C₃₋₈ cycloalkyl;
alternatively, R² and R³, together with the atoms to which they are attached, form 5- to 6-membered nitrogen-containing heterocyclyl, wherein the 5- to 6-membered nitrogen-containing heterocyclyl is optionally substituted with 0 to 3 identical or different substituents selected from deuterium, halogen and C₁₋₆ alkyl;
preferably,
R³ is independently selected from hydrogen and propynyl;
alternatively, R² and R³, together with the atoms to which they are attached, form 5- to 6-membered nitrogen-containing heterocyclyl, wherein the 5- to 6-membered nitrogen-containing heterocyclyl is optionally substituted with 0 to 3 identical or different substituents selected from C₁₋₆ alkyl.

7. The compound according to any one of claims 1 to 6, a tautomer, stereoisomer or pharmaceutically acceptable salt thereof, wherein R^{4a} and R^{4b} are each independently selected from hydrogen, halogen and C₁₋₆ alkyl, wherein the C₁₋₆ alkyl is optionally substituted with 0 to 3 identical or different substituents selected from deuterium, amino, hydroxy, C₁₋₃ alkyl, C₁₋₃ alkoxy, C₁₋₃ haloalkyl, C₁₋₃ haloalkoxy and halogen;
alternatively, R^{4a} and R^{4b}, together with the carbon atoms to which they are attached, form C₃₋₄ cycloalkyl or 3- to 4-membered heterocycloalkyl; wherein the heterocycloalkyl contains 1 heteroatom selected from O, N, C(=O) and S; the C₃₋₄ cycloalkyl and 3- to 4-membered heterocycloalkyl are each optionally substituted with 0 to 6 identical or different substituents selected from deuterium and halogen;
alternatively, R³ and R^{4a}, together with the atoms to which they are attached, form 3- to 7-membered nitrogen-containing heterocyclyl; wherein the nitrogen-containing heterocyclyl contains 1 to 3 N atoms and 0 to 3 heteroatoms selected from O and C(=O); wherein the 3- to 7-membered heterocyclyl is optionally substituted with 0 to 6 identical or different substituents selected from deuterium, amino, hydroxy, C₁₋₃ alkyl, C₁₋₃ alkoxy, C₁₋₃ haloalkyl, C₁₋₃ haloalkoxy and halogen; R^{4b} is selected from hydrogen, halogen and C₁₋₆ alkyl, wherein the C₁₋₆ alkyl is optionally substituted with 0 to 3 identical or different substituents selected from deuterium, amino, hydroxy, C₁₋₃ alkyl, C₁₋₃ alkoxy, C₁₋₃ haloalkyl, C₁₋₃ haloalkoxy and halogen;
preferably,
R^{4a} and R^{4b} are each independently selected from hydrogen, fluorine and methyl;
alternatively, R^{4a} and R^{4b}, together with the carbon atoms to which they are attached, form C₃₋₄ cycloalkyl or 3- to 4-membered heterocycloalkyl; wherein the heterocycloalkyl contains 1 O atom; the C₃₋₄ cycloalkyl and 3- to 4-membered heterocycloalkyl are each optionally substituted with 0 to 6 identical or different halogens;
alternatively, R³ and R^{4a}, together with the atoms to which they are attached, form 3- to 7-membered nitrogen-containing heterocyclyl; wherein the nitrogen-containing heterocyclyl contains 1 to 3 N atoms, and 0 to 3 heteroatoms selected from O and C(=O); the 3- to 7-membered nitrogen-containing heterocyclyl is optionally substituted with 0 to 6 identical or different substituents selected from amino, hydroxy, C₁₋₃ alkyl and halogen; R^{4b} is selected from hydrogen, fluorine and methyl;
alternatively, R³ and R^{4a}, together with the atoms to which they are attached, form 3- to 6-membered nitrogen-containing heterocyclyl; wherein the nitrogen-containing heterocyclyl contains 1 N atom; the 3- to 6-membered nitrogen-containing heterocyclyl is optionally substituted with 0 to 3 identical or different substituents selected from amino, hydroxy, C₁₋₃ alkyl and halogen;
alternatively, R³ and R^{4a}, together with the atoms to which they are attached, form 3- to 5-membered nitrogen-containing heterocyclyl; wherein the nitrogen-containing heterocyclyl contains 1 N atom; the 3- to 5-membered nitrogen-containing heterocyclyl is optionally substituted with 0 to 2 identical or different substituents selected from amino, hydroxy, C₁₋₃ alkyl and halogen;
alternatively, R³ and R^{4a}, together with the atoms to which they are attached, form 4-membered nitrogen-containing heterocyclyl; wherein the nitrogen-containing heterocyclyl contains 1 N atom; the 4-membered nitrogen-containing heterocyclyl is optionally substituted with 0 to 2 identical or different substituents selected from amino, hydroxy, C₁₋₃ alkyl and halogen;
more preferably,
R^{4a} and R^{4b} are each independently selected from hydrogen, fluorine and methyl; alternatively, R^{4a} and R^{4b}, together with the carbon atoms to which they are attached, form cyclopropyl, cyclobutyl or a 4-membered oxacycle; wherein the cyclopropyl, cyclobutyl and 4-membered oxacycle are each optionally substituted with 0 to 6 fluorines;
alternatively, R³ and R^{4a}, together with the atoms to which they are attached, form nitrogen-containing 3-membered monocyclic heterocyclyl, 4-membered monocyclic heterocyclyl, 5-membered monocyclic heterocyclyl, 6-membered monocyclic heterocyclyl, 7-membered monocyclic heterocyclyl, 5-membered spiro heterocyclyl, 5-membered bridged heterocyclyl, 6-membered spiro heterocyclyl, 6-membered bridged heterocyclyl, 6-membered fused heterocyclyl, 7-membered spiro heterocyclyl, 7-membered bridged heterocyclyl or 7-membered fused heterocyclyl; wherein the nitrogen-containing heterocyclyl contains 1 to 3 N atoms and 0 to 3 heteroatoms selected from O and C(=O); the nitrogen-containing heterocyclyl is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 3-membered monocyclic heterocyclyl, 4-membered monocyclic heterocyclyl, 5-membered monocyclic heterocyclyl, 6-membered monocyclic heterocyclyl or 7-membered monocyclic heterocyclyl; wherein the nitrogen-containing heterocyclyl contains 1 to 3 N atoms and 0 to 3 heteroatoms selected from O and C(=O); the nitrogen-containing heterocyclyl is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 3-membered monocyclic heterocyclyl, 4-membered monocyclic heterocyclyl, 5-membered monocyclic heterocyclyl, or 6-membered monocyclic heterocyclyl; wherein the nitrogen-containing heterocyclyl contains 1 to 3 N atoms and 0 to 3 heteroatoms selected from O and C(=O); the nitrogen-containing heterocyclyl is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 3-membered monocyclic heterocyclyl, 4-membered monocyclic heterocyclyl, 5-membered monocyclic heterocyclyl, or 6-membered monocyclic heterocyclyl; wherein the nitrogen-containing heterocyclyl contains 1 N atom; the nitrogen-containing heterocyclyl is optionally substituted with 0 to 3 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 3-membered monocyclic heterocyclyl, 4-membered monocyclic heterocyclyl, or 5-membered monocyclic heterocyclyl; wherein the nitrogen-containing heterocyclyl contains 1 N atom; the nitrogen-containing heterocyclyl is optionally substituted with 0 to 2 identical or different substituents selected from amino, hydroxy, methyl, ethyl, propyl, isopropyl, fluorine, chlorine and bromine; R^{4b} is selected from hydrogen, fluorine and methyl;
alternatively, R³ and R^{4a}, together with the atoms to which they are attached, form nitrogen-containing 4-membered monocyclic heterocyclyl; wherein the nitrogen-containing heterocyclyl contains 1 N atom; the nitrogen-containing heterocyclyl is optionally substituted with 0 to 2 identical or different substituents selected from amino, hydroxy, methyl, and fluorine; R^{4b} is selected from hydrogen, fluorine or methyl;
further preferably,
R^{4a} and R^{4b} are each independently selected from hydrogen, fluorine and methyl; alternatively, R^{4a} and R^{4b}, together with the carbon atoms to which they are attached, form cyclopropyl, cyclobutyl or a 4-membered oxacycle; wherein the cyclopropyl, cyclobutyl and 4-membered oxacycle are each optionally substituted with 0 to 6 fluorines;
alternatively, R³ and R^{4a}, together with the atoms to which they are attached, form a nitrogen-containing heterocyclyl group selected from 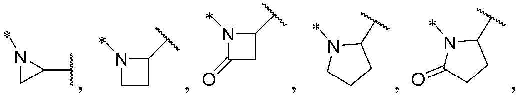 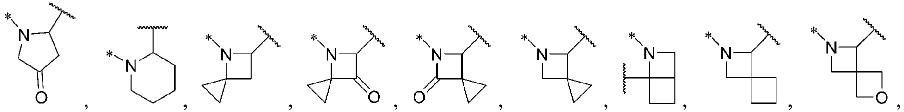 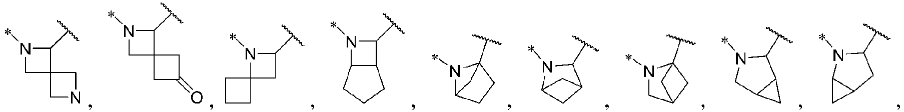 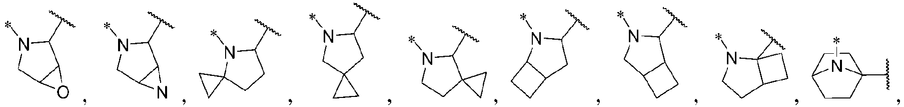 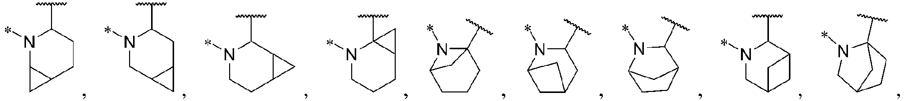 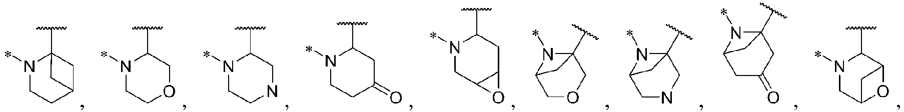 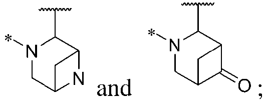 wherein 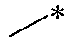 denotes the linkage to the Y end and 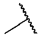 denotes the linkage to C(O)NH₂; each of the nitrogen-containing heterocyclyl groups is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl;
still further preferably,
R^{4a} and R^{4b} are each independently selected from hydrogen, fluorine and methyl;
alternatively, R^{4a} and R^{4b}, together with the carbon atoms to which they are attached, form cyclopropyl, cyclobutyl or a 4-membered oxacycle; wherein the cyclopropyl, cyclobutyl and 4-membered oxacycle are each optionally substituted with 0 to 6 fluorines;
alternatively, R³ and R^{4a}, together with the atoms to which they are attached, form a nitrogen-containing heterocyclyl group selected from 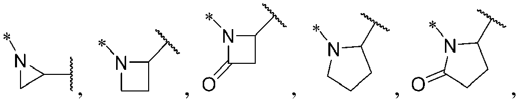 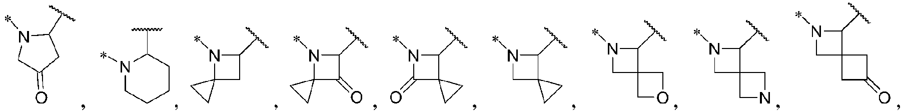 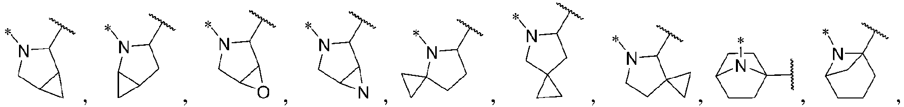 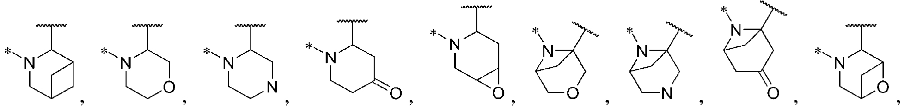 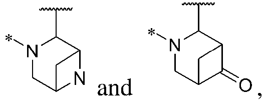 etc.; wherein 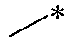 denotes the linkage to the Y end and 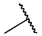 denotes the linkage to the C(O)NH₂ end; each of the nitrogen-containing heterocyclyl groups is optionally substituted with 0 to 6 identical or different substituents selected from amino, hydroxy, methyl, ethyl, propyl, isopropyl, fluorine, chlorine and bromine; R^{4b} is selected from hydrogen, fluorine and methyl;
still further preferably,
R^{4a} and R^{4b} are each independently selected from hydrogen, fluorine and methyl;
alternatively, R^{4a} and R^{4b}, together with the carbon atoms to which they are attached, form cyclopropyl, cyclobutyl or a 4-membered oxacycle; wherein the cyclopropyl, cyclobutyl and 4-membered oxacycle are each optionally substituted with 0 to 6 fluorines;
alternatively, R³ and R^{4a}, together with the atoms to which they are attached, form 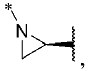 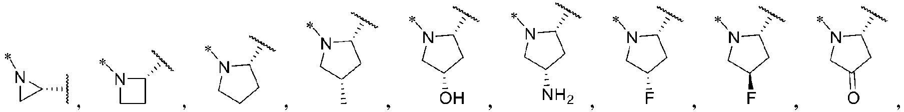 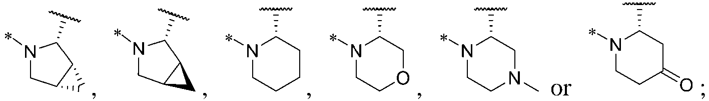 wherein 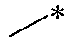 denotes the linkage to the Y end and 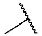 denotes the linkage to the C(O)NH₂ end; R^{4b} is selected from hydrogen, fluorine and methyl;
alternatively, R³ and R^{4a}, together with the atoms to which they are attached, form 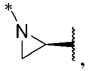 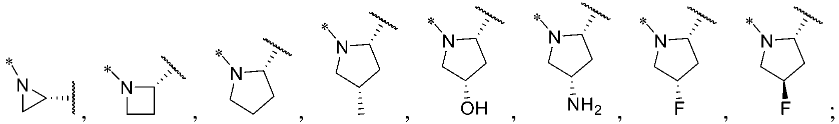 wherein --* denotes the linkage to the Y end and 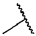 denotes the linkage to the C(O)NH₂ end; R^{4b} is selected from hydrogen, fluorine and methyl;
alternatively, R³ and R^{4a}, together with the atoms to which they are attached, form 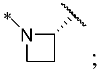 wherein 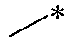 denotes the linkage to the Y end and 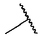 denotes the linkage to the C(O)NH₂ end; R^{4b} is selected from hydrogen, fluorine and methyl;
alternatively, R³ and R^{4a}, together with the atoms to which they are attached, form 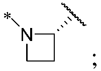 wherein 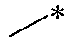 denotes the linkage to the Y end and 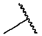 denotes the linkage to the C(O)NH₂ end; R^{4b} is selected from hydrogen.

8. A compound having the structural formulas shown below, a tautomer, stereoisomer or pharmaceutically acceptable salt thereof:

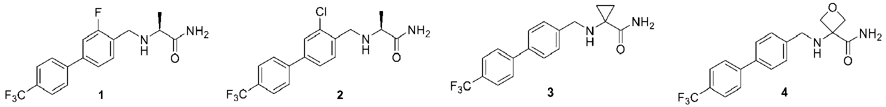

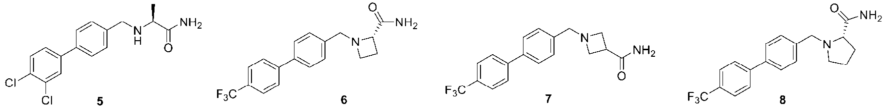

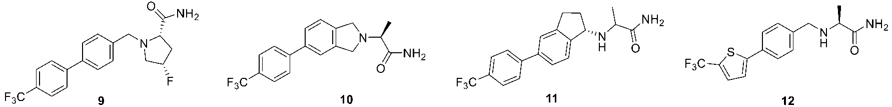

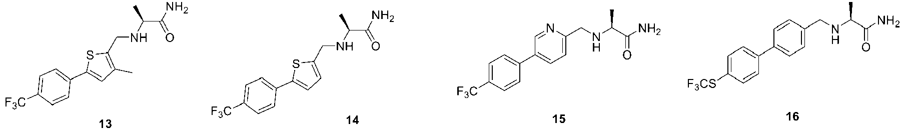

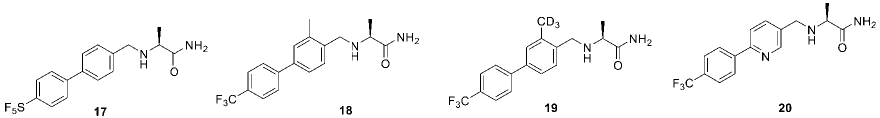

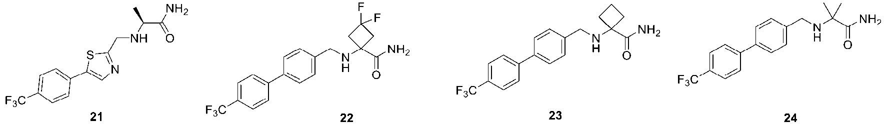

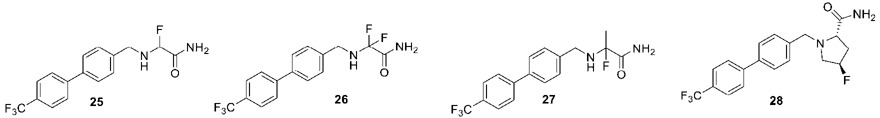

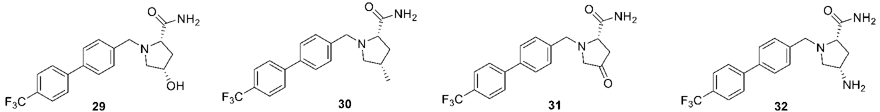

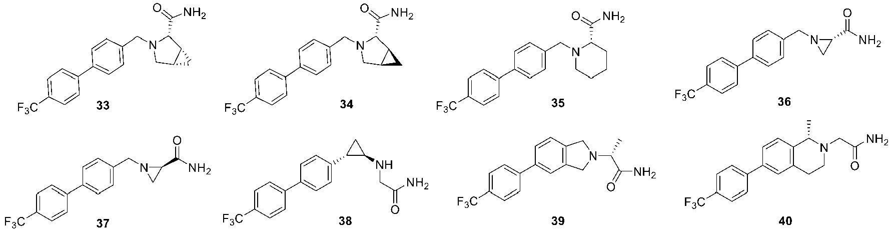

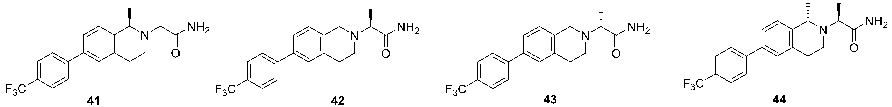

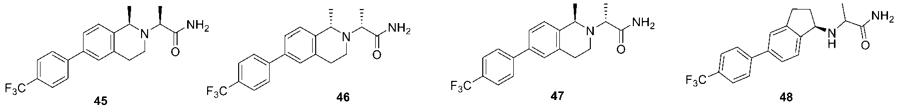

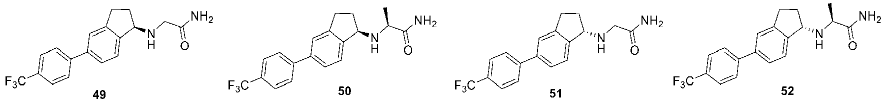

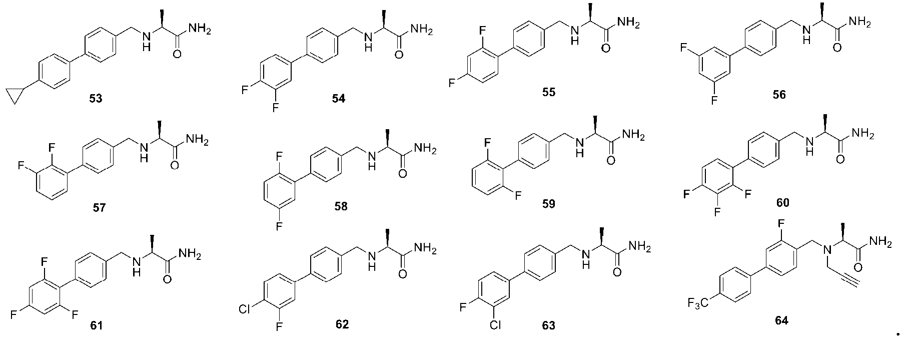

9. The compound according to any one of claims 1 to 8, a tautomer, stereoisomer or pharmaceutically acceptable salt thereof, wherein the pharmaceutically acceptable salt is selected from: methanesulfonate salts.

10. A pharmaceutical composition, comprising the compound according to any one of claims 1 to 9, a tautomer, stereoisomer or pharmaceutically acceptable salt thereof, and optionally further comprising a pharmaceutically acceptable carrier.

11. Use of the compound according to any one of claims 1 to 9, a tautomer, stereoisomer or pharmaceutically acceptable salt thereof, or the pharmaceutical composition according to claim 10 in the preparation of a medicament for diseases or disorders associated with overexpression of monoamine oxidase-B (MAO-B).

12. The use according to claim 11, wherein the medicament is used for preventing, ameliorating and/or treating diseases or disorders related to memory, cognition, mood, neurodegeneration, obesity and the like.

13. The use according to claim 11 or 12, wherein the diseases or disorders are selected from the group consisting of Alzheimer's disease, depression, stroke, Parkinson's disease, hypertension and obesity.

14. A medicament, comprising the compound according to any one of claims 1 to 9, a tautomer, stereoisomer or pharmaceutically acceptable salt thereof, wherein the medicament is for preventing, ameliorating and/or treating diseases or disorders associated with overexpression of monoamine oxidase-B (MAO-B);
preferably, the diseases or disorders associated with overexpression of monoamine oxidase-B (MAO-B) are those related to memory, cognition, mood, neurodegeneration and obesity; more preferably: Alzheimer's disease, depression, stroke, Parkinson's disease, hypertension or obesity.

15. A method for preventing, ameliorating and/or treating diseases or disorders associated with overexpression of monoamine oxidase-B (MAO-B), comprising administering to a patient a therapeutically effective amount of the compound according to any one of claims 1 to 9, a tautomer, stereoisomer or pharmaceutically acceptable salt thereof, or the pharmaceutical composition according to claim 10; preferably, the diseases or disorders associated with overexpression of monoamine oxidase-B (MAO-B) are those related to memory, cognition, mood, neurodegeneration, obesity and the like, preferably: Alzheimer's disease, depression, stroke, Parkinson's disease, hypertension or obesity.
